# EUROPEAN PATENT APPLICATION

(11) **EP 4 137 096 A1**
(43) Date of publication of application: **22.02.2023**
(21) Application number: 20930176.1
(22) Date of filing: 07.04.2020
(51) Int. Cl.: A61F 2/26, B29C 41/00, B29C 41/02

(54) **IMPLANTABLE PROSTHETIC DEVICE FOR PRODUCING A PENILE ERECTION IN MEN WITH ERECTILE DYSFUNCTION AND PRODUCTION METHOD THEREOF**

(71) Applicant: Pontificia Universidad Católica de Chile, Santiago (CL)
(72) Inventor: MARCONI TORO, Marcelo Carlos, Santiago (CL); GONZÁLEZ RAMOS, Alberto Alejandro, Santiago (CL); CARO PINTO, Ivan Alberto, Santiago (CL)
(74) Representative: Ipsilon
(86) International application number: PCT/CL2020/050036
(87) International publication number: WO 2021/203212

(57) **Abstract**

Disclosed is a prosthetic device (1) that can be implanted in the corpora cavernosa of a penis with erectile dysfunction, and the production method thereof, the device being formed by two cylinders that can be inflated with a fluid. The device can be implanted in a wide range of patients according to penis length and optimises the use of a low volume of the fluid to achieve a functional erection. The device comprises a variable-length erectable portion (10) formed by a chamber (100) that can be pressurised with a low volume of fluid and which is formed between an expandable membrane (300) and an axially extendable and foldable longitudinal rib (200). The membrane (300) and the rib (200) extend between a distal tip element (400) and an intermediate connector (500) from which a shortenable anchoring rod (600) emerges, the rib (200) being a single piece formed by a variable-length distal section (206), an intermediate section (205) that is foldable and extendable in an axial direction, and a proximal section (204) comprising optimised means for the continuous lateral supply of the fluid to the pressurisable chamber (100). The device (1) also has an integrated unit (700) for storing and pressurising the fluid, which can be implanted in the scrotum.

## Description

The present invention generally relates to medical devices implantable within the body of a patient, and more particularly, relates to an inflatable prosthetic device implantable within the *corpus cavernosum* of the penis in order to cause erection of the organ in men who suffer from severe erectile dysfunction that do not respond to first and second line treatments, where the device optimizes the use of a low volume of fluid.

The Application also refers to the production procedure of the prosthetic device which allows various sizes of the device to be produced in a simplified and economical way.

### PRIOR ART DESCRIPTION

Erectile dysfunction (ED) is a sexual dysfunction characterized by the inability to develop or maintain a penile erection during sexual activity. In a man, dilation of the arteries leading to the *corpus cavernosum* of the penis causes congestion with consequent enlargement and stiffness of the penis while the enlargement restricts penile blood flow through the venous channels. Various psychological and physiological causes (for example, smoking, obesity, long-term diabetes, spinal cord damage, neuropathies, atherosclerosis or damage after pelvic surgery) are associated with ED in men.

Regardless of the cause, erectile dysfunction has a very important effect on the quality of life and the psychological state of both the subject who suffers from it and his partner. In some cases where the patient does not respond to conventional therapy, surgical implantation of a penile prosthesis may be the only practical means of remedying impotence.

When talking about solutions for erectile dysfunction, there is usually a distinction between three lines of medical responses: a first line that is related to making changes in habits, psychological therapy or the prescription of oral medications, among which are some hormone replacements. A second line of treatment aims at slightly more invasive actions, such as the use of vacuum devices that cause erections or injections of vasoactive substances that are applied directly to the penis in order to generate erections. Then there are the third line treatments which are directly invasive but with guaranteed results, including penile prostheses or vascular surgery which are procedures used when the patient does not respond to first or second line solutions.

The present invention points to this third line of solutions specifically referring to a prosthesis. Historically, prostheses have been broadly classified into two types, non inflatable prostheses and inflatable prostheses.

Non-inflatable prostheses are further divided into fixed inclination prostheses and malleable prostheses; the former are the oldest solutions, they are rigid rods that bring some inclination in a predetermined manner between a proximal portion that is anchored to the patient's body and a distal portion that remains in the pendular area of the penis; a good example of this type of fixed inclination implant can be seen described in the US Patent document US3893456 by Heyer Schulte Corp published on 07/08/1975.

Non-inflatable malleable prostheses, on the other hand, normally comprise a semi-rigid body with some portion or means that allow it to be folded manually, generally held in a downward position and manually folded upwards to obtain an erection prior to sexual intercourse. An example of this malleable type is described in document US4665902 by Bard Inc. published on 05/19/1987 which shows an implant with two rigid end portions between which an intermediate portion with a bellows structure is arranged allowing the implant folding.

The advantages of non-inflatable penile prostheses, malleable or not, lie in their ease of use since the user can erect the penis very quickly by simply bending it up or down; they have a lower failure rate because they are simple structures and have a low production cost.

However, some of its disadvantages include the semi-rigid appearance of the penis at all times although when the patient does not want an erection it can be manually bent downwards, basically it maintains a permanent erection, so it is very unnatural in state of flaccidity. They also imply a greater risk of discomfort and erosion in the penile tissue due to the constant rigidity; implantation requires very large incisions and its measurements, being fixed, require the production of a wide range of sizes and thicknesses in order to be useful to the greatest number of patients who need it.

Inflatable prostheses are broadly divided into three-part and two-part prostheses; in the case of the former, they comprise inflatable cylinders that are arranged in the cavernous body of the penis, a reservoir with the filling fluid that is normally implanted in the abdomen, and a pump that drives the bidirectional transfer of a fluid between the reservoir and the cylinders, where normally the pump is installed in the scrotum. An example of this type is described in Patent document US4566446 by Mentor Corp published on 01/28/1986.

One of the main disadvantages of these three-part prostheses is the number of pieces that must be implanted inside the patient, since the prostheses themselves include two cylinders, each one implantable in the cavernous bodies of the penis, from each of them a duct emerges that communicates with two pumping devices implanted in the scrotum and they, in turn, communicate through other ducts with the fluid reservoir located in the abdominal area.

Two-piece prostheses comprise two inflatable cylinders connected to a duct that communicates with a unit that integrates the pump with the reservoir into a single element, thus reducing the number of parts to be installed. An example of this type of implant is shown in document US4009711 by Uson, published on 03/01/1977.

The main advantages of inflatable systems, whether they are of two or three parts are given because they allow better regulation of the size of the prosthesis in operation, being able to deflate the cylinders when an erection is not desired, so that it gives a slightly more natural appearance in state of flaccidity compared to non-inflatable prostheses that remain permanently erect, and another advantage is that their implantation allows for smaller incisions because the cylindrical pieces are introduced into the *corpus cavernosum* in a deflated state.

However, the main disadvantages they have is that there is a higher failure rate due to the number of parts and joints specially with self-inflation errors; on the other hand, by including pieces that go into the abdomen, the scrotum and the penis as such, it implies a more invasive implantation increasing the possibility of infections; and perhaps one of the biggest disadvantages is related to the amount of fluid that must be handled to achieve a functional erection, since in most of these implants at least 20 ml. per cylinder is handled, 40 ml. total; each standard pump displaces 4 mL, so inflating cylinders requires at least 10 pumps.

Within this group of inflatable prostheses, solutions have been sought that try to combine the advantage of the single piece, as occurs in the malleable ones, and the advantage of the change in the physical state of the penis when it is erect and when it is flaccid so that it looks and feel more natural. From this, a group of solutions has been derived where an attempt is made to incorporate the reservoir and the pump mechanism in the same implantable cylinder, as described in Patent document US4360010 by Medical Eng. Corp. published on 11/23/1982, where in the same cylinder of the implant there is a bladder-shaped proximal zone that extends to the proximal end of the anchorage, within that zone there is a mechanical pumping means that allows the transfer of fluid to and from the distal portion that resides in the pendular portion of the penis.

It has been found that these types of solutions do not work well, since the user has complex access to the pumping or drive mechanism; it also happens that the cylinder body, being completely hollow, requires a larger amount of fluid that is difficult to store in the back of the same cylinder; the amount of fluid that it can actually contain, due to the small size of the integrated reservoir, is not enough to cause an effective erection.

In the state of the art it is possible to observe developments of inflatable prostheses that address problems that have to do with the flexibility of the cylinder, specially related to the shape that the penis adopts in a state of flaccidity, since a recurrent problem is that in inflatable implants with a hollow interior, such as the one described in Patent document US4881530 by Medical Eng. Corp, published on 11/21/1989, it happens that once the cylinders are emptied to cancel the erection, they acquire a functionally inadequate shape and an aspect that is unnatural, because when the cylinder is emptied it folds downwards, generating a transverse fold and sometimes the edges of the fold show through the skin, damaging the tissue due to the constant friction that occurs with that edge; in addition, it acquires a very unnatural appearance in a state of flaccidity, being noticed as an empty sheath.

Another of the disadvantages that this type of implant entails is that, being hollow, they need a large amount of fluid to achieve an erection, which undesirably results in a good erection being difficult due to the weight that the penis acquires; in addition to the inconvenience that implies for a user the number of times the pump must be actuated to be able to transfer that large amount of fluid from the reservoir to the cylinders, resulting in an excessive manipulation of the scrotum compression where the fluid driver pump is normally implanted. As mentioned in a previous point, in these systems at least 20 ml. per cylinder is handled, which implies 40 ml. total; each standard pump displaces 4 mL, so inflating cylinders requires at least 10 pumps. This generates psychological effects in the patient, since many feel ashamed or anxious because the excessive manipulation necessary to achieve an erection is not natural and interrupts the natural rhythm of the intimate moment of the couple.

In search of overcoming the problem of the high quantity of fluid necessary to produce the erection in inflatable prostheses, together with the problem of the unnatural appearance of the penis in a state of flaccidity, it is possible to find in the state of the art a line of devices with inflatable cylinders that have an inner longitudinal core-type piece that occupies a considerable volume inside the cylinder cavity, so that the amount of fluid necessary to cause an erection decreases ostensibly.

Some of them have this longitudinal core that is made of a single piece integrated with the anchoring stem, but they have a different diameter or different hardness between a more rigid proximal anchoring portion and a more flexible distal portion that occupies the pendular area of the penis, such as the prostheses that are described in documents US4558693 by Harvey Lash 1985 and in US4201202 by Medical Engineering 1980. In both cases the filling of the inflatable cylinder occurs directly in a chamber that is generated around the longitudinal core, at which joins a flexible conduit which in turn is attached to a reservoir. In some cases, as in the last cited patent, the cylinder or the layer that generates the inflatable chamber is normally made of a flexible and thin material that allows its pressurization without the expansion being excessive to the level of damaging tissues, being a type of textile material coated with silicone, which being impermeable allows the required elasticity.

The main disadvantage of this type of solutions just described is that they do not ensure a supply of fluid that is free of obstruction in the coupling area of the flexible conduit that comes from the reservoir, which can easily become obstructed by compression of the surrounding tissue or by the normal folds that are generated under the base of the penis in a flaccid state, requiring greater pumping pressure to overcome the obstruction of the fluid feeding duct.

Solutions for this are described in other documents, where the prostheses comprise that piece in the form of a longitudinal core with the surrounding inflatable space that is filled with fluid and the proximal anchoring element, where said proximal anchoring element includes an inner conduit that communicates with the inflatable space and communicates with a flexible tube that comes from the reservoir and pumping system, so that said inflatable space does not have a direct connection coupling with the fluid supply but is connected by this additional piece that is usually more rigid and also works as an anchor. An example of this is seen in the Patent US5067485 by Mentor Corp 1991 and in the Patent US7390296 by AMS Research Corp 2008.

In the first one, this proximal anchoring piece with an internal channel that helps to resolve a possible collapse of the filling coupling area is seen, however it has the disadvantage that the core-shaped piece is hollow and must also be filled to cause the erection, therefore, brings about the described problem of the use of a large amount of fluid and weight of the pendulum portion. In the case of the second patent cited above, although it includes among all its embodiments one where its core element is solid and therefore, the surrounding space must only be filled with fluid, doing so through this proximal piece that has an interior channel and that functions as an anchor, this solution has the problem that the longitudinal core-type element is not attached to the base at its proximal end, the central element is pushed forward by the fluid that enters axially from said anchor element, so it requires a large internal pressurization that prevents the prosthesis from folding downwards in said area where the central element is not attached to the base; furthermore, to achieve radial expansion as well, ultimately it needs to handle a lot more fluid inside.

Other solutions that also include a longitudinal core-type element, incorporate the reservoir in the same cylinder, especially in the proximal anchorage area, as seen in US4399812 by Edgar D. Whitehead 1983, which with the inclusion of an intermediate inner chamber comprising valve means, allows the passage of fluid from the proximal reservoir to the intermediate and from there to a peripheral chamber surrounding the longitudinal core element.

There are also other types of solutions in devices with longitudinal core-type elements, where they directly seek to eliminate the reservoir, as described in US4,532,920 by Medical Engineering 1985, where a peripheral chamber is pressurized by a fluid that is manually injected into the point of the core; in this case, it is possible to completely eliminate a reservoir with fluid, however, this solution has a great disadvantage which is the need that every time the patient wishes to have an erection the fluid must be injected with a syringe directly into the tissue of the glans until reaching the device and filling it.

The advance that technology has shown since the incorporation of a longitudinal core or core element within the cylinders of the prostheses has meant an important contribution in the reduction of necessary fluid and of the appropriate mechanisms for its pumping as well as it has also contributed significantly on the problem of the unnatural appearance that the penis takes on in a state of flaccidity, since with these solutions a state of relaxation is achieved without the bending folds of the cylinder showing through or the organ looking like an empty sheath.

Of all these types of solutions that can be found in the state of the art, although they include important advantages, it is possible to notice that none of them attempts to solve all these problems in the most comprehensive way possible and where, in addition, the problem of the adaptation or adjustment of the prosthesis size in a variable margin of the anatomy of the penis to be treated is considered, not only with respect to the initial width and length, or the length or width that it can acquire with the incorporation of the prosthesis, but also that there are other variables that are also important which together make it difficult to standardize a size that works for most patients, such as the relationship between the size of the glans and the rest of the penis specially in prostheses that have actuation mechanisms in said zone; the length-width relationship as such of the penis where it has been found that some prostheses respond well to the width of the penis but not to its length or vice versa; the anatomy of the pelvic area where the prostheses are anchored and the distance of the scrotal sacs from the body of the penis.

There are many factors that affect the choice of the best prosthesis size to install in a patient so normally each prosthesis model is manufactured in several sizes at the same time and they are usually marketed together as a prosthesis set, so that the surgeon can count on this range of sizes at the time of implantation surgery; but certainly this situation makes the whole procedure more expensive.

Of the known technologies there are some that only allow the length of the anchoring proximal piece of the prosthesis to be adjusted, which are manufactured with a loose length whose end can be cut by the surgeon at the time of implantation, as described in Patents US4201202 and US4532920 already cited, the latter even mentioning the provision of annular cuts or grooves that facilitate the removal of the excess portion.

In view of the above, the need for a type of penile prosthesis to deal with severe erectile dysfunction becomes evident, one that not only addresses the different technical problems mentioned above, such as the optimization of fluid pressurization, the effectiveness of the erection and the unnatural appearance in a flaccid state but also addresses the production problem that normally makes the device more expensive, since all existing solutions lack a manufacturing solution that allows at least some parts not to require special tooling for each size to which the prosthesis must be manufactured.

### DESCRIPTION OF THE INVENTION

The present invention relates to a prosthetic device of the type composed of inflatable and implantable cylinders within the *corpus cavernosum* of the penis in order to cause erection of the organ in men suffering from severe erectile dysfunction who do not respond to first and second line treatments. Additionally, the invention also refers to the production procedure of said device which allows to reduce costs associated with a multiplicity of tooling.

One of the main objectives of the invention is to provide a prosthetic device that optimizes the use of a low volume of fluid achieving a functional erection in a short time and with little manipulation by the patient.

Another of the main objectives of the invention is to provide a prosthetic device that ensures the filling of the cylinders without being obstructed in the area of fluid entry either by the contraction of the cylinder itself or by compression generated by the surrounding tissue.

Still another of the objectives of the present invention is to provide a prosthetic device that, by requiring less pressurization fluid, it can dispense with a large reservoir in addition to the pump to house a large amount of fluid so it can have a system of only two parts, of the type that integrates the reservoir with the pumping system and with a valve in a single implantable element in the scrotal sacs.

With the foregoing, the problem of excessive manipulation by the patient to achieve an erection is also being solved, avoiding having to perform numerous pumping gestures manipulating his scrotal area, given that the present invention needs to move little fluid and thanks to the conjunction with optimized characteristics, it only requires one pump to achieve the erection of the organ.

An additional objective of the invention is to provide a prosthetic device that gives the penis a more natural appearance when it is in a flaccid state, in the sense that it does not remain permanently erect or remains contracted and folded downwards forming folds that erode the skin with constant rubbing, but allows a natural downward curvature with a natural decrease in total volume.

Another objective of the invention is to provide a prosthetic device that facilitates its implantation in the least invasive way possible being able to make small incisions for the introduction of the cylinder, since this in addition to being able to be installed in a deflated state, allows the most rigid parts to be completely folded before being incorporated into the *corpora cavernosa* of the penis.

Regarding the production procedure, its main objective is to conceive the aforementioned prosthetic device, with its feasibility of offering several lengths, but with a lower production cost since it does not need to have special tooling for each required length.

The present prosthetic device is of the type that is made up of two cylinders that are inflatable with a fluid, each one implantable in each of the *corpora cavernosa* of the penis, which are linked to an integrated fluid storage and pressurization unit that is placed in the scrotal sacs and that delivers fluid to and from each of the device inflatable cylinders.

Each inflatable cylinder comprises an erectable distal portion, an anchoring stem arranged at the proximal end of the device, and an intermediate connector that communicates said erectable portion with the anchoring stem and with the integrated fluid storage and pressurization unit.

The erectable distal portion allows optimizing the use of a low volume of pressurized fluid to cause penile erection in a short time and with little manipulation by the patients ensuring its filling without being obstructed or overstrained in the area of fluid entry by the contraction of the same cylinder or by compression of the surrounding tissue. It also allows the flaccid penis to acquire a natural appearance not like an empty sheath.

This distal portion of the cylinder is composed of a chamber that is pressurizable with a low volume of fluid from the aforementioned integrated storage and pressurization unit where the chamber is a cylindrical space that is formed between an expandable membrane in the axial and radial directions, and a longitudinal internal nerve which is flexible, axially extensible and foldable.

This nerve and the expandable membrane extend longitudinally between the intermediate connector and a point element disposed at the distal end of the cylinder.

The nerve causes an axial extension of the penis and gives it resistance to compression in an erect state; plays an important role in reducing the fluid required to achieve an erection since this nerve occupies a large part of the chamber where said chamber is capable of containing and keeping the fluid pressurized inside. Whereas in a flaccid state, when the chamber is not pressurized with fluid, the collapsible nerve filling its interior space allows the penis to retain a natural looking downward curvature and turgor. On the other hand, the nerve also influences the optimization of the filling of the chamber since it comprises optimized means of dispensing the fluid which allow its continuous passage and without resistance.

The nerve is formed of a robust elongated body of strong but flexible material, such as a biocompatible elastomeric material, which is preferably a medical grade silicone polymer. Its body has a rounded section, it is preferably cylindrical in shape with an outer diameter less than the diameter of the chamber; the body is defined by a continuous outer surface, a straight proximal end that attaches to the intermediate connector, and a distal end that attaches to the distal point element of the device.

The nerve is in one single piece, and three axial sections can be distinguished defined by a proximal section with a hollow interior, an intermediate section that is foldable and axially extensible, and a distal section of variable length.

The proximal section of the nerve is a cylindrical section with a mantle of constant diameter and the optimized means for the continuous lateral dispensing of the fluid towards the pressurizable chamber are arranged in it. These means consist of an inner axial channel, through which the fluid is conducted from the intermediate connector towards the pressurizable chamber to cause the erection of said erectable portion.

The inner axial channel has an open proximal end for the fluid inlet/outlet, it extends straight to an internal end point that is pointed, where it branches forming lateral channels with an inclined path with respect to an imaginary axial axis of the nerve.

The lateral channels flow out into corresponding openings diametrically opposite each other that appear on the sides of the mantle of said proximal section, these openings being elliptical in outline; while the internal point where the branching of the channel occurs has a pointed projection that facilitates the division of the flow of the fluid so that its supply is equally towards each lateral opening.

This lateral position of the openings arranged punctually in the horizontal axis of the nerve and not arranged on the upper and/or lower face, has several advantages: on the one hand it allows said chamber to begin filling from the area of the penis base to drive the fluid load progressively from the top towards the distal end and thus avoid lifting the weight of the fluid from the critical end of the bending moment, reducing the pressure when pumping manually; it also favours the constant passage of the fluid towards the chamber, because if they were on the vertical axis, for example on the upper side, the flow would have to fight against the force of gravity to emerge, requiring greater pumping pressure; it would also happen that the openings would be more exposed to compression and obstruction caused by the same membrane that may collapse and stick to the openings due to the vacuum effect, making it especially difficult to start pumping the fluid, as it would happen if the opening were on the underside where it is more likely to become clogged with accumulated and folded tissue in the flaccid state.

Instead, as these openings are on a horizontal axis and appear on the sides of the nerve, they allow the exit of the fluid to be more continuous and to be carried out with less effort specially at the beginning of the filling, favouring the generation of an expansive pressure of the membrane that prevents obstruction of the openings.

Filling with pressurized fluid before branching, this inner channel of the nerve offers a slight resistance that generates a first erection impulse from the base of the penis and, as the fluid passes with constant pressure into the chamber, it is capable of continue erecting the rest of the nerve by unfolding its corrugated area, which supports the point of the prosthesis to maintain a specific length in order to present the penis with a significant size for the patient.

The channels and openings have a suitable diameter to favour the conduction of the fluid, if they are very narrow, the friction alters the hydraulics of the fluid increasing the pressure when pumping and, in turn, any particle of some external element could obstruct the outlet channel. If they are very large, it weakens the silicone nerve leaving it prone to cutting due to the traction generated by the same tensile pressure and the stress caused by the transition from flaccidity to erection. The lateral channels extend diagonally because they improve the hydraulics in the exit of the fluid when pumping, diagonally they generate that the fluid has less friction when leaving and that it suffers less change of direction in its exit, because if these channels were at right angles perpendicular along the nerve, they would cause the fluid to generate vortices when it collides with the membrane at the moment of exit altering the continuous exit of the fluid and requiring a higher pumping pressure.

Thus, the diameter, inclination and lateral location of the openings favour rapid inflation and deflation of the chamber which is very important for the patient and his partner, since it improves the spontaneity of sexual intercourse.

The fact that this proximal section of the nerve is hollow allows it to be made more flexible in part, since it is through itself that the nerve is fixed to the connector, thus the curvature of the penis in a state of flaccidity is even more natural, since it begins to curve from its base.

The other axial section that is part of the nerve is the intermediate section, it extends continuously after the proximal section and its main characteristic is that it presents a decrease in its mass, giving this portion a condition of high flexibility and elasticity that allows it to fold and expand axially to almost twice its initial length, pulled in part by the point element and the membrane's own expansion; meanwhile, the folding that it is able to achieve generates a kind of hinged cylinder like a closed compass that allows it to be introduced through a small incision at the time of its implantation in the penis and to move it through the proximal and distal *corpora cavernosa.*

In a first embodiment of the nerve this intermediate section has an axial core of reduced diameter with a plurality of annular ribs forming a corrugation that allows the core to fold but still maintaining a radially resistant structure.

In another alternative embodiment of the nerve said intermediate section has a microperforated structure that weakens the portion, lightens it, and allows it to fold and also to extend axially.

In yet another embodiment of the nerve said intermediate section has a surface with successive radial depressions forming an accordion shaped portion that also allows the nerve to fold and its axial extension.

Finally, the third axial section of the nerve corresponds to the distal section extended from the intermediate section which is preferably solid, cylindrical, with a straight mantle and it is attached to the point element of the device through its distal end.

In a preferred embodiment of the nerve the proximal section and the intermediate section always have a single average length while the distal section has an original length that exceeds its final length, where its distal end can be freely cut to define the full length of the nerve and thus obtain various formats of different lengths during production.

In an alternative embodiment of the nerve it has at least three different total lengths, defining a prosthesis with a longer length, one with a medium length and another with a shorter length where the proximal section always has a single average length while the intermediate and the distal section are of different lengths, among which the preferred condition is that the length of the intermediate section represents between 24% and 30% of the total length of the nerve.

In these alternative embodiments of the nerve where the total length is predetermined to certain lengths and therefore, its distal end cannot be cut, present a perimeter recess in said distal end which allows it to couple in an opening present in the point element of the device which is independent of a slot where the extensible membrane is coupled.

The hollow proximal section of the nerve will always have a single predetermined length, since being at the base and containing the means of dispensing the fluid does not allow its end to be cut, since its length affects the distance that the lateral openings take with respect to the base and would also modify the location of the collapsible intermediate portion with respect to the base of the penis.

The other component element of the erectable portion of the device is the extensible tubular membrane that coaxially surrounds the nerve, this is a textile membrane, formed by a tubular fabric with a continuous structure without longitudinal seams which is embedded in medical silicone that allows generate high pressure in the chamber and provides rigidity ensuring its extensible characteristic under controlled sealing and impermeability conditions. The membrane itself is a pneumatic structure of which textile composite material with silicone allows this membrane to have a controlled and regular deformation throughout its length avoiding superficial aneurysms.

The membrane expands radially and longitudinally due to two variables that act systemically, the structure of the tubular tissue and the elastic condition of the silicone in which the tissue is embedded, where this structure can be formed by an orthogonal woof where both the longitudinal and the concentric fibres present a normally corrugated state that allows their expansion in both directions when requested; alternatively, said fabric can also be made up of a mesh of fibres arranged diagonally that allow the membrane to expand in both directions.

This membrane comprises a cylindrical tubular body with an open distal end, an open proximal end, an inner surface and an outer surface that define a thickness of the membrane which is preferably 1mm. It is attached to the intermediate connector through its proximal end and is attached to the point element through its distal end.

It acts by expanding in a radial and axial direction due to the effect of pressurization in the chamber, it contains the fluid and allows it to be pressurized thanks to its ability to regulate its own expansion; it must be considered that excessive expansion can cause pain and damage to the surrounding tissue, although in some way, the *tunica albuginea* of the penis which is a natural elastic fibrous sheath that forms an irregular intertwined network that covers the *corpus cavernosum* of the penis, also acts containing the expansion of the prosthesis.

This capacity of the membrane to limit its expansion in order to provide the necessary rigidity of the chamber and at the same time of the erection, is given by the relationship of thickness, length and the elastomeric material from which it is made which due to its elastic memory will always tend to oppose the internal pressure of expansion limiting excessive growth.

As mentioned previously, the pressurizable chamber is formed by a cylindrical space that is coaxial to the nerve radially comprised between the outer surface of said nerve and the inner surface of the tubular membrane, and has a proximal limit axially provided by the connector, and a distal limit provided by the point element of the device; where this chamber has a width given by the radial distance between said nerve and said membrane which preferably corresponds to 1.5 millimetres.

The pressurizable chamber begins to fill or to empty from its proximal end where it is in communication with the lateral openings of the nerve which, in turn, communicate it with the integrated fluid storage and pressurization unit.

The point element of the erectable portion of the device is solid, made of elastomeric material such as medical grade silicone, it has a rounded distal end in the shape of a cap that is housed in the portion of the penis glans and a proximal portion with a straight edge comprising a central axial opening where it jointly receives the distal end of the nerve and the distal end of the tubular membrane. This element also includes a small transverse perforation that is useful at the time of implantation of the prosthesis which serves to pull the prosthesis towards the distal end.

In an alternative embodiment of the point element, it comprises the same central axial opening where it receives the distal end of the nerve and additionally comprises an outer annular groove coaxial to the central opening where it receives the distal end of the tubular membrane.

The intermediate connector that is part of the device is a rigid piece, also made of medical silicone, with a distal end that has a central cavity where the proximal end of the central nerve is coupled, an annular groove coaxial to the central cavity where the end proximal of the tubular membrane is coupled, it has an opposite end with a central recess where the anchoring stem of the device is coupled.

The intermediate connector also has an internal conduit that is formed by a front axial part and a curved rear part where the axial part has an open end that connects with the central cavity that receives the nerve while the curved part of the channel flows out in a lower opening. This inner conduit allows the passage of the fluid between the integrated storage and pressurization unit and the proximal portion of the nerve that supplies the fluid towards the pressurizable chamber.

The integrated fluid storage and pressurization unit comprises a flexible reservoir that stores the fluid, a pressure relief valve in line with the flexible reservoir, and a pair of flexible conduits that extend from the valve to each of the connectors on each cylinder. The flexible conduit is coupled to the intermediate connector at its lower opening, such that those flexible conduits communicate the valve and flexible reservoir with the erectable portion of the device through the intermediate connector. The valve is located in close proximity to the reservoir allowing both to be implanted as a single unit in the scrotal sacs.

Said unit comprises a single flexible reservoir and a single valve to supply both cylinders of the device, for which the reservoir is a flexible lateral compression structure with bellows-shaped walls, which operates both as a reservoir and as a pumping mechanism, this being allowed by the flexibility of the material which can also be medical grade silicone and by the bellows shape of the reservoir, as it optimizes the pumping pulsations generated by compression achieving that with few compressions, preferably just one, the fluid can be rapidly mobilized and pressurized within the chamber.

In an alternative embodiment, the manual pump may be replaced by an electronic micro pump that would take up the same space as the tank.

The pressure relief valve establishes controlled fluid communication between the erectable portion of the device and said bellows shaped reservoir; while said valve is connected on one side to the reservoir in the form of a bellows and on the other, it is connected to a pair of flexible conduits that transfer the fluid from the reservoir to each of the erectable portions of the device.

The bellows shaped reservoir contains a sufficient amount of fluid to pressurize each of the device's chambers where said chambers, thanks to the material of the extensible membrane and the characteristics of the nerve, can be pressurized with a low amount of fluid that goes from 3 to 5 ml. for each chamber achieving axial rigidity and weight support that is similar to a conventional inflatable prosthesis, but instead of using 36 ml. of total fluid it only requires a total amount between 6 to 10 ml. of fluid which is divided between both cylinders.

The anchoring stem of the device is preferably placed in the root segment of the *corpus cavernosum,* its body is preferably cylindrical with an end for attachment to the connector and an opposite end that can be cut to adjust the total length of the cylinder at the time of implantation. It comprises a series of spaced annular grooves that demarcate points that facilitate its cutting.

The nerve, the point element, the intermediate connector, the anchoring stem and the integrated storage unit are made of biocompatible elastomeric material, such as a medical grade silicone polymer that can be applied in different thicknesses and hardness for each one of the elements.

The different joints that occur between the elements, such as the joint between the membrane and nerve with the point element and with the intermediate connector, as well as the joint between the anchoring stem and the connector are preferably made with a suitable silicone adhesive that forms firm and watertight joints.

The membrane, being a textile material embedded in silicone allows its wall to be thin, extensible in radial and axial directions, but equally resistant, weighing less than the weight of a cylinder made entirely of silicone without a textile structure.

The low weight of the membrane, in combination with the low weight that the nerve also has given that its proximal section is hollow, and its intermediate section has a decreased mass, means that the erectable portion of the cylinder can be pressurized adequately with much less fluid than is needed for conventional cylinders.

The special combination between the functional and structural characteristics of the membrane, the nerve and the integrated storage and pressurization unit allow a pressurization of the chamber with a low volume of fluid that does not exceed 5 ml, stiffens and erects the penis in little time and with little pumping effort by the patient, achieving a good effect with just one pump.

The relatively small volume chamber can normally hold residual fluid and thus be further pressurized with a small amount of fluid from the reservoir to harden the implant and generate a penile erection; while this residual fluid present in the chamber together with the nerve favours the natural appearance of the penis in a flaccid state, because although it shortens, thins and folds downwards, the chamber does not collapse due to vacuum to the point of generating a fold because the central nerve and the residual fluid prevent it.

With regard to the production method of the prosthetic device just described, it allows various sizes of the device to be produced in a simplified and economical manner. All its components are independently manufactured parts that can then be combined and assembled during the assembly stage where the device is also pre-filled. Specifically, the cylinders each comprise a set of five pieces which are: the anchoring stem, the connector, the nerve, the membrane and the point element; where at least two of these pieces can be manufactured with an indeterminate continuous length that can be freely cut at the time of the device assembly, which allows the production to be of lower cost by not having to count on special tooling to produce each length of said at least two pieces.

These at least two pieces that can be manufactured with an undetermined continuous length are the tubular membrane and the anchoring stem where the membrane can be produced as a continuous tubular sleeve that is then cut into sections of a suitable length to match the length of the nerve while the anchoring stem can be produced as an extruded or moulded rod of indefinite length which is cut to certain lengths to form part of the device, this stem being the only piece that allows the final cutting of its proximal end at the time of the implantation surgery of the prosthesis in the patient which allows to generate a final adjustment of the length; the rest of the device is assembled, sealed and pre-filled with the fluid in its production stage.

The nerve, the connector, the stem and the point element are preferably manufactured by moulding the silicone in a specific die-making for each shape.

The nerve cannot be produced with an indeterminate continuous length like the membrane because it has three axial sections that are different from each other, however, in a first embodiment of the production procedure the nerve is manufactured with a single length where its distal section has an exceeded length with a margin that can later be cut at its distal end in order to obtain different formats of shorter length while its foldable intermediate section has an average length that allows optimal operation in any of the defined final lengths. Thus, the production of the nerve from a single length with a wide margin allows it to be moulded in a single matrix that is used for all lengths avoiding the multiplication of matrices of different sizes to obtain each of the prostheses of different sizes to cover a relatively wide range of patients with different penis sizes. Achieving with this a substantial saving in time and production costs.

In a preferred example of the production procedure, the longest manufacturing length of the nerve is 16 centimetres, from which, by cutting its distal end, cut ribs of, for example, 14 cms, 12 cms, 10 cms and a minimum of 8 cms can be obtained, where each of these measures allows conceiving prostheses of 22, 20, 18, 16 and 14 total centimetres, respectively.

Each of these lengths of the nerve that are obtained, the membrane that has been manufactured with an indefinite length or manufactured with a length greater than which at least five or ten prostheses can be produced, is dimensioned in pieces of the same length as the nerve obtained; the manufacture of the membrane in these conditions, whether of an indefinite length or much longer, allows the total production of the prosthesis to dispense with a multiplication of die-cutting of specific dimensions for each length of membrane that is needed. Achieving with this a new substantial saving in time and production costs.

In this first embodiment of the production procedure, the point element is manufactured with a single central cavity at its straight end in which the distal end of the cut nerve and the distal end of the tubular membrane, are spliced together. So that the diameter of said cavity is slightly greater than the diameter of the distal end of the nerve allowing both pieces to couple tightly inside it to be sealed in a watertight manner.

In a second embodiment of the production procedure, said nerve is manufactured in at least three predetermined lengths: a maximum length, an intermediate one and a minimum one where the length of its folding intermediate section also has a determined length to adjust proportionally to the total length of the nerve, this in order that the prosthesis erection capacity, rigidity, turgidity or deflection are not affected.

For example, in the case of a shorter prosthesis said folding section should not be less than 22 mm. long so as not to affect the erection capacity and in turn, allow the bending of the prosthesis when introducing it into the *corpora cavernosa* and it cannot be greater than 30 mm. in the case of longer prostheses because it would reduce the rigidity of the nerve, especially when obtaining turgor in a state of flaccidity.

In this second production embodiment, where the nerve is manufactured with pre-determined lengths, although it does not represent the same level of savings in manufacturing compared to the first embodiment of the procedure, it still contributes to die-cutting savings since the extensible membrane continues to be produced in the same way of an indeterminate length that is then dimensioned according to the length of the moulded nerve as is done with the anchoring stem, which is always manufactured of an indeterminate or exceeded length in order to obtain a single longer length at least five or ten minor stems mountable in the same number of prostheses produced.

In this second embodiment of the device production procedure where the distal end of the nerve does not need to be cut, it comprises at said end a perimeter recess to fit into the point element in a central opening that is independent of an annular groove where the membrane is coupled.

Thus, said point element would also have a second embodiment where, in addition to having the central cavity where the lowered end of the nerve fits it has an annular groove coaxial to the central one where the distal end of the membrane is spliced.

The diameter of the proximal end of the nerve coincides with the diameter of the central cavity of the connector where it is tightly sealed when the device is assembled, generating a continuous channel between said inner channel of the connector and the inner axial channel of the nerve. Meanwhile, the proximal end of the membrane fits and seals itself to the annular groove coaxial to the central cavity of the connector generating a slight separation between the nerve and the membrane in said proximal area which favours the start and continuity of the chamber filling, since the membrane is not attached to the outer surface of the nerve just in the area where the openings through which the fluid passes are located. The diameter of the anchor stem coincides with the diameter of the central recess of the proximal end of the connector where it is also mounted and sealed in the assembly process of the device.

As it is possible to see, in either of the two production embodiments the membrane and the anchoring stem are always manufactured in continuous lengths that can then be cut into suitable lengths to adapt to each of the different measurements of the device.

Thus, the device production procedure according to a first embodiment, comprises the following steps:
a) moulding the nerve (200) of a single predefined length with the exceeded length distal section (206);
b) moulding the point element (400) with a single axial opening (404) at its proximal end (402);
c) moulding the intermediate connector (500);
d) manufacturing the membrane (300) with a constant diameter and thickness, and an indeterminate length as a tubular sleeve;
e) moulding the anchoring stem (600) of a determined diameter and indeterminate length;
f) defining the final length of the nerve (200) cutting its distal end (201);
g) sizing and cutting a piece of membrane (300) in a length according to the final length of the cut nerve (200) defining a distal end (301) and a proximal end (302) of said piece of membrane (300) already cut;
h) mounting, joining and sealing the joints of all the pieces together;
i) arranging the integrated storage and pressurization unit (700) with a predefined amount of internal fluid according to the final size of the prosthesis and joining its flexible conduits (703) with each of the intermediate connectors (500).

When moulding the intermediate connector, the central cavity of the distal end must have a diameter matching the outer diameter of the proximal end of the nerve, its coaxial groove must have a size matching the cross-section of the piece of tubular membrane, its central recess in the proximal face must have a diameter matching the diameter of the connecting stem, and its inner channel must have a lower opening with a diameter matching the upper end of the flexible conduit of the integrated storage and pressurization unit.

By mounting, joining and sealing all the pieces together the distal end of the nerve and the distal end of the membrane are coupled together and sealed within the central axial opening of the point element; the proximal end of the nerve is coupled and sealed in the central cavity of the connector while the proximal end of the membrane is coupled and sealed to the annular groove coaxial to the central cavity of the connector; the distal end of the anchoring stem is coupled and sealed within the central cavity of the proximal end of the connector; and the upper end of the flexible conduit of the integrated unit is coupled and sealed to the lower opening of the connector.

The nerve, the intermediate connector, the point element, the anchoring stem and the flexible reservoir of the integrated storage and pressurization unit are moulded in biocompatible elastomeric material, preferably a medical grade silicone polymer in suitable matrices for each part.

The production procedure of the prosthetic device according to a second embodiment, comprises the steps of:
a) moulding the nerve (200) according to at least three different predefined lengths, each with the intermediate section (205) of a length proportional to the total length and with its distal section (206) comprising a perimeter recess (207) at its point;
b) moulding the point element (400) having a central axial opening (404) and an outer annular groove (405);
c) moulding the intermediate connector (500);
d) manufacturing the membrane (300) with a constant diameter and thickness and an indeterminate length as a tubular sleeve;
e) moulding the anchoring stem (600) of a determined diameter and indeterminate length;
f) sizing and cutting a piece of membrane (300) in length according to the length of the moulded nerve (200), defining a distal end (301) and a proximal end (302) of said piece of membrane (300);
g) mounting, joining and sealing the joints of all the pieces together.
h) arranging the integrated storage and pressurization unit (700) with a predefined amount of internal fluid according to the final size of each prosthesis and joining its flexible conduits (703) with each of the intermediate connectors (500).

In this second embodiment of the method, the at least three different predefined lengths for the nerve correspond to a shorter length, a longer length and an intermediate length. Where preferably the shortest length is 8 centimetres, the intermediate length is 12 centimetres and the longest length is 16 centimetres; meanwhile, the membrane is preferably manufactured in a thickness of approximately 1mm.

In this second embodiment of the method, when the point element is moulded, the central axial opening has a diameter equivalent to the diameter of the lowered end of the nerve and its outer annular groove has a cross section that coincides with the cross section of the membrane.

In this second embodiment of the procedure, when moulding the intermediate connector its central cavity at the distal end has a diameter that matches the outer diameter of the proximal end of the nerve; its coaxial groove is sized to match the cross section of the membrane; its central recess on the proximal face has a diameter that coincides with the diameter of the anchoring stem; and the lower opening of its inner channel has a diameter that coincides with the upper end of the flexible conduit of the integrated storage and pressurization unit.

By mounting, joining and sealing all the pieces together, in this second embodiment of the procedure the lowered distal end of the nerve is coupled and sealed within the central axial opening of the point element; the distal end of the membrane is coupled and sealed within the axial annular groove at the central opening of the point element; the proximal end of the nerve is coupled and sealed in the central cavity of the connector while the proximal end of the membrane is coupled and sealed to the annular groove coaxial to the central cavity of the connector; the distal end of the anchoring stem is coupled and sealed within the central cavity of the proximal end of the connector; and the upper end of the flexible conduit of the integrated unit is coupled and sealed to the lower opening of the connector.

The nerve, the intermediate connector, the point element, the anchoring stem and the flexible reservoir of the integrated storage and pressurization unit are moulded in biocompatible elastomeric material preferably a medical grade silicone polymer in suitable matrices for each part.

### DESCRIPTION OF THE FIGURES

For the achievement of the objectives, the invention can be performed in the form illustrated in the accompanying drawings; however, the drawings are only illustrative and do not limit the scope of the invention and may acquire multiple implementations as long as they are under a common inventive concept. Thus, a detailed description of the invention will be carried out together with the Figures that are an integral part of this presentation, where:
Figure 1 shows a side view of the prosthetic device, in an erect state.
Figure 2 shows a front isometric view of the prosthetic device.
Figure 3 shows a side elevation view of the nerve of the prosthetic device.
Figure 4a shows an enlarged sectional front view of the proximal nerve portion of the prosthetic device.
Figure 4b shows a bottom plan view of the proximal portion of the prosthetic device nerve.
Figure 5 shows a lateral elevation view of the prosthetic device nerve where its intermediate portion stands out.
Figure 6 shows a side elevation view of the prosthetic device nerve, where a first embodiment of its distal portion stands out.
Figure 7 shows a side elevation view of the prosthetic device nerve where a second embodiment of its distal portion stands out.
Figure 8 shows a sectional front elevation view of the extensible membrane of the prosthetic device.
Figure 9 shows a sectional side elevation view of a complete cylinder of the prosthetic device.
Figure 10 shows a top plan view of a complete cylinder of the prosthetic device in a flaccid state.
Figure 11 shows an enlarged sectional side view of a first embodiment of the point element of the prosthetic device.
Figure 12 shows a sectional side view of a connection detail between the point element of Figure 11 with the nerve and the membrane.
Figure 13 shows an enlarged sectional side view of a second embodiment of the point element of the prosthetic device.
Figure 14 shows a sectional side view of a connection detail between the point element of Figure 13 with the nerve and the membrane.
Figure 15 shows a sectional side view of the intermediate connector.
Figure 16 shows a sectional side view of the junction of the intermediate connector with the nerve, the membrane and the anchoring stem.
Figure 17 shows a side view of a cylinder of the prosthetic device in the intermediate erection state.
Figure 18 shows a top front isometric view of the prosthetic device in an erect state.
Figure 19 shows the set of cylinder parts that are made of silicone.
Figure 20 shows a side view of the complete prosthetic device in a completely flaccid state.
Figure 21 shows a side view of the complete prosthetic device in an intermediate state of erection.
Figure 22 shows a side view of the complete prosthetic device in an erect state.
Figure 23 shows a sectional side view of a first embodiment of the prosthetic device nerve.
Figure 24 shows a side view of the extensible membrane of indeterminate length in the stage of dimensioning and cutting its final length.
Figure 25 shows a partial sectional side view of the nerve, the membrane, the intermediate connector and the anchoring stem facing each other for assembly.
Figure 26 shows a sectional side view of a connection detail of the first embodiment of the point element with the first embodiment of the prosthetic device nerve.
Figure 27 shows a partial sectional side view of the nerve, the membrane, the intermediate connector and the anchoring stem already assembled together.
Figure 28a shows a partial sectional side view of a second embodiment of the nerve according to a smaller size production.
Figure 28b shows a partial sectional side view of a second embodiment of the nerve according to an intermediate size production.
Figure 28c shows a partial sectional side view of a second embodiment of the nerve according to a larger size production.
Figure 29 shows a side view of the extensible membrane of indeterminate length in the stage of dimensioning and cutting its final length.
Figure 30 shows a sectional side view of a connection detail of the second embodiment of the point element with the second embodiment of the prosthetic device nerve.

### DETAILED DESCRIPTION OF THE INVENTION

The invention refers to a prosthetic device (1) that can be implanted inside the cavernous body of a penis with erectile dysfunction and to its production method where the device is of the type formed by two cylinders that are inflatable with a fluid, allowing it to be implanted in a wide range of patients according to the length of their penis and optimizes the use of a low volume of fluid in order to achieve a functional erection.

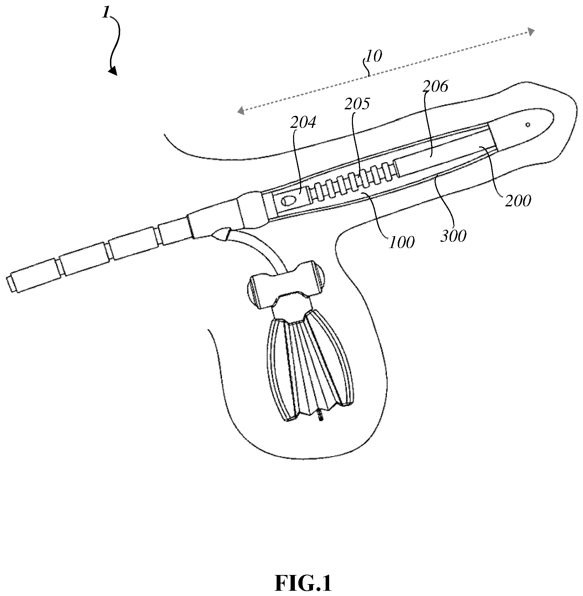

As it can be seen in FIG. 1, the device (1) comprises an erectable portion (10) of variable length, composed of a pressurizable chamber (100) with a low volume of fluid formed between an expandable membrane (300) and an axially extensible and foldable longitudinal internal nerve (200) where said nerve (200) is in one piece formed by a distal section (206) of variable length, an intermediate section (205) that is foldable and extensible in the axial direction, and a proximal section (204) comprising optimized means for the continuous lateral supply of the fluid towards the pressurizable chamber (100).

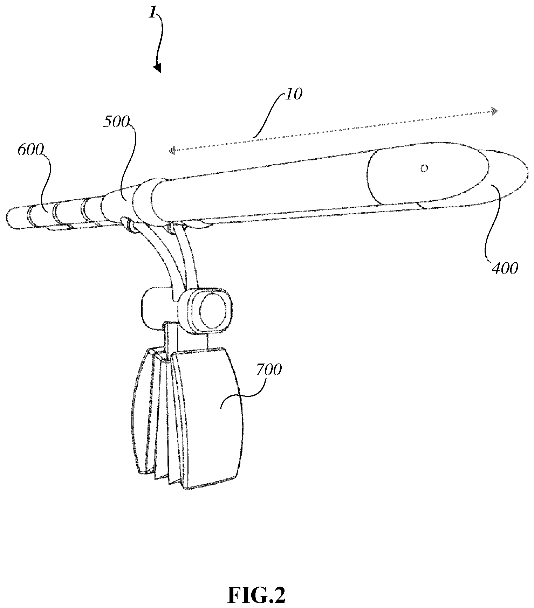

Looking now at FIG. 2, it is possible to see that the erectable portion (10) of the device (1) extends between a distal point element (400) and an intermediate connector (500) which also connects to a cuttable anchoring stem (600) and an integrated unit (700) for storage and pressurization of the implantable fluid in the scrotal sacs.

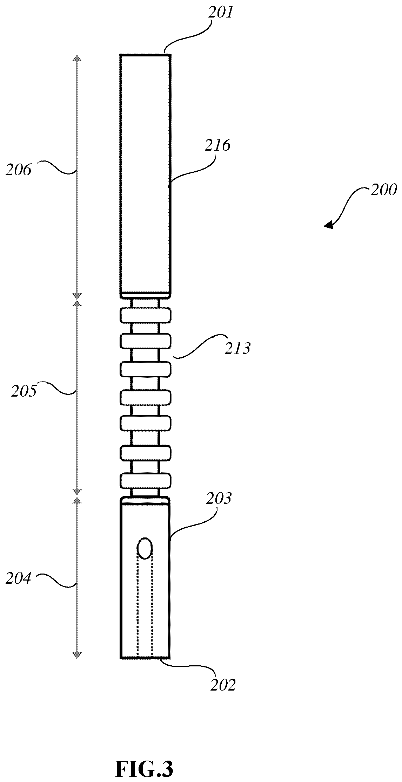

As best seen in FIG. 3, the nerve (200) is formed by a robust longitudinal body with a continuous outer surface (203), a distal end (201) and a proximal end (202) between which the proximal (204), intermediate (205) and distal (206) sections extend, where the proximal section (204) is partially hollow, the intermediate section (205) has a reduction (213) in its mass and the distal section (206) has a mantle (216) of variable length.

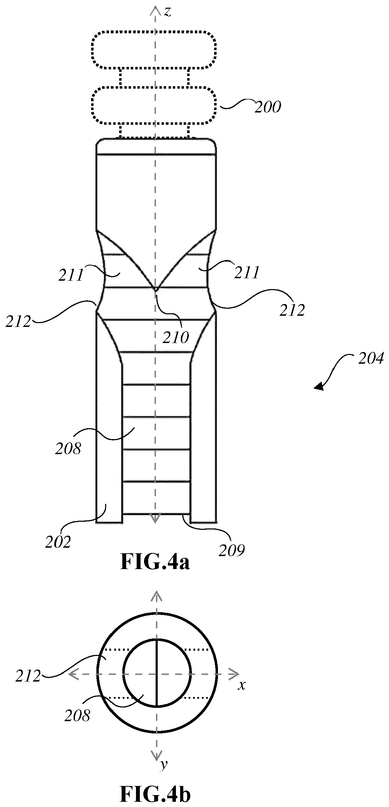

In relation to the aforementioned optimized means of continuous lateral delivery of the fluid, these are illustrated more clearly in FIG. 4a where an increased detail of the proximal portion (204) of the nerve (200) can be appreciated, the means are composed of an internal axial channel (208) in said proximal portion (204) where said channel has an open end (209) through which the fluid enters and which coincides with the proximal end (202) of the nerve, the axial channel (208) it extends to an opposite internal end (210) which is pointed; from this area, the axial channel branches laterally according to a horizontal axis (x) of the nerve, as shown in FIG. 4b forming two lateral channels (211) that flow into corresponding lateral openings (212) with an elliptical contour through which the fluid exits out of the nerve, said lateral channels (211) having an inclined path with respect to an axial axis (z) of the nerve (200).

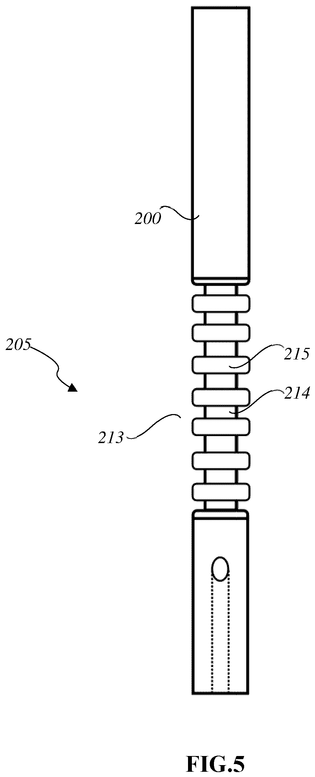

As for the intermediate section (205) of the nerve (200) which is foldable and extensible in the axial direction, according to a first embodiment shown in FIG. 5, it has a decrease (213) in its mass that allows that zone acts as a collapsible and axially extensible portion; it has an axial core (214) of smaller diameter than the rest of the nerve (200) and comprises a spaced plurality of annular ribs (215) forming a corrugation that allows the folding of that area and in turn, allows its axial extension.

In an alternative embodiment (not illustrated), said intermediate section (205) has a microperforated structure that weakens the portion and allows it to be folded and axially extended. Similarly, in another alternative embodiment (not illustrated) said intermediate section (205) has a surface with successive radial depressions forming an accordion-shaped portion that allows its folding and axial extension.

Regarding the length of said intermediate section (205), in one embodiment of the nerve this section has a single length; while in another embodiment of the nerve the intermediate section (205) has different preset lengths.

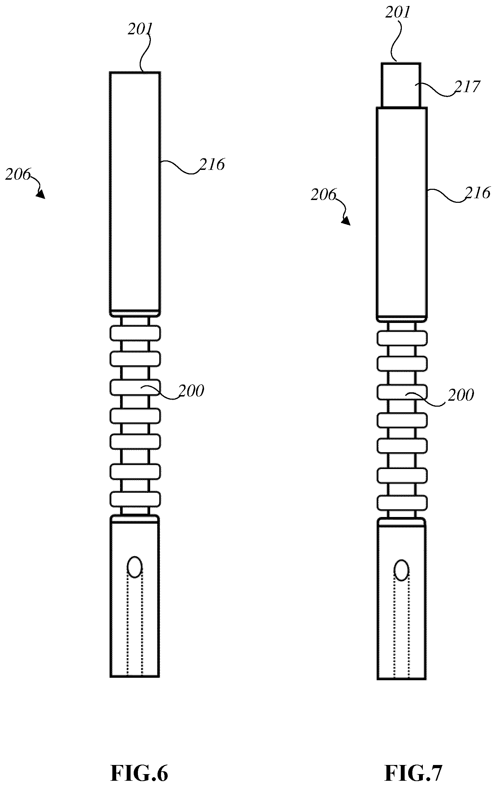

In relation to the distal section (206) of the nerve (200), in a preferred embodiment shown in FIG. 6 it has a straight mantle (216) of distal end (201) that can be freely cut to define different shorter lengths of the nerve and thus assembling different prostheses of different lengths. And in an alternative embodiment of said distal section (206), as seen in FIG. 7, it has a straight mantle (216) with a distal perimeter recess (217) that generates a smaller diameter with respect to the mantle (216) where in this alternative embodiment the distal section (206) is not cuttable, but is produced in different preset lengths.

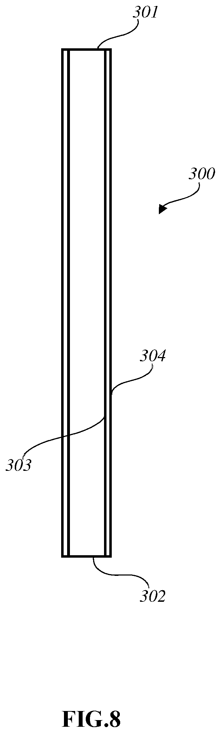

The expandable membrane (300) is a textile membrane formed by a continuous tubular fabric without longitudinal seam embedded in medical silicone that allows high pressure and rigidity, ensuring its expandable characteristic under sealing and impermeability conditions; referring to the illustration of FIG. 8 the membrane (300) comprises an open distal end (301), an open proximal end (302), an inner surface (303), and an outer surface (304). Where said membrane (300), being of a textile structure, comprises an orthogonal woof where both the longitudinal and the concentric fibres present a normally corrugated state that allows the expansion of the membrane in both directions; in an alternative embodiment it comprises a diagonal woof that also allows the membrane to expand in both directions.

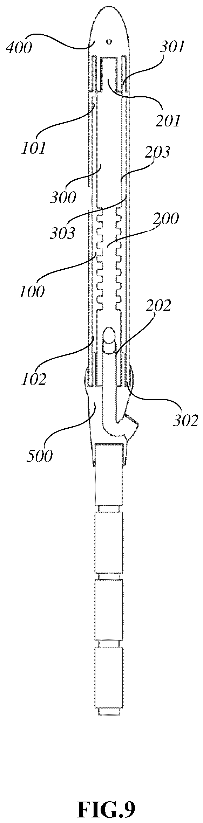

As best shown in FIG. 9, the membrane (300) is attached to the intermediate connector (500) through its proximal end (302) and is attached to the point element (400) through its distal end (301), as well as the nerve (200) is attached to the intermediate connector (500) through its proximal end (202) and is attached to the point element (400) through its distal end (201). Between the membrane (300) and the nerve (200) the pressurizable chamber (100) is formed, describing a cylindrical space comprised radially between the outer surface (203) of the nerve (200) and the inner surface (303) of the membrane (300), and axially it has a distal zone (101) that limits with the distal point element (400) and a proximal zone (102) that limits with the intermediate connector (500).

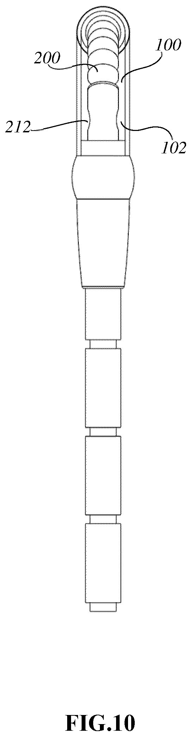

As illustrated in FIG. 10, the pressurizable chamber (100) begins to fill or to empty from its proximal area (102) with the fluid that emerges or enters through the lateral openings (212) of the nerve (200).

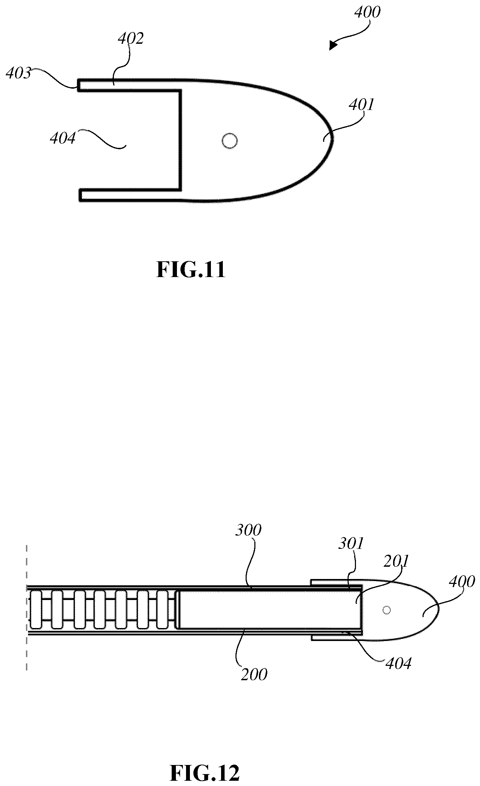

Referring to FIG. 11, in a preferred embodiment of the point element (400) said element is solid of elastomeric material, it has a rounded distal end (401) and a proximal portion (402) with a straight edge (403) comprising a central axial opening (404) where it jointly and sealedly receives the distal end (201) of the nerve (200) and the distal end (301) of the membrane (300), as seen in FIG. 12.

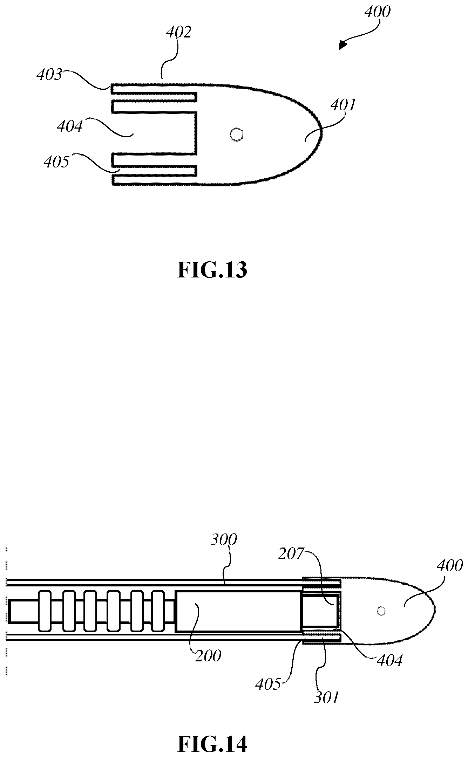

In an alternative embodiment of the point element (400) shown in FIG. 13, it is made of solid elastomeric material, has a rounded distal end (401) and a proximal portion (402) with a straight edge (403) comprising a central axial opening (404) where it receives the recess (207) of the nerve (200) and further comprises an outer annular groove (405) coaxial to the central opening (404) where it receives the distal end (301) of the membrane (300), as seen in FIG. 14.

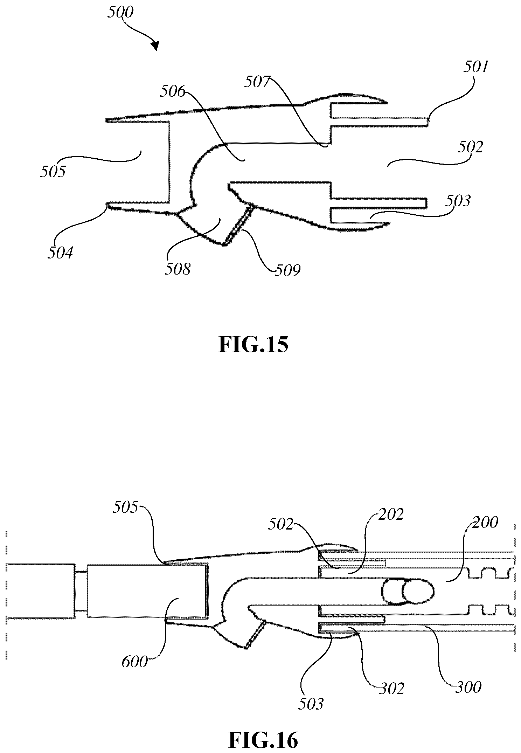

Taking into consideration the content of FIG. 15, the intermediate connector (500) is a solid piece with a distal end (501) that has a central cavity (502), an annular groove (503) coaxial to the central cavity (502), a proximal end (504) with a central recess (505) and an inner channel (506). Said inner channel (506) is curved, it has an open front end (507) that connects with the central cavity (502) and a lower curved end (508) that flows into a lower opening (509).

And as best exemplified in FIG. 16, the central cavity (502) of the connector (500) is coupled with the proximal end (202) of the nerve (200); the annular groove (503) is coupled with the proximal end (302) of the membrane (300); while its central recess (505) is coupled with the anchoring stem (600).

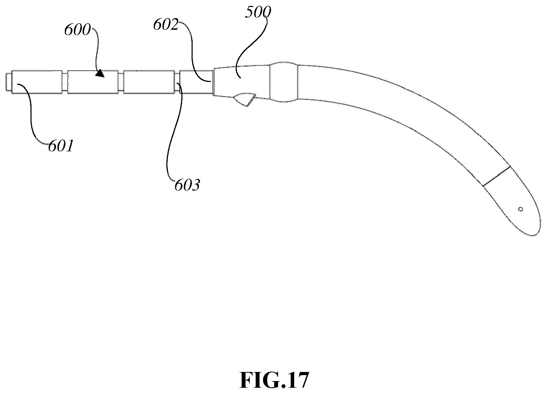

Said anchoring stem (600), as seen in FIG. 17, is a preferably cylindrical body with a rear end (601) that can be cut and a front end (602) through which it is attached to the intermediate connector (500); it comprises a series of annular grooves (603) regularly spaced from each other that determine cut points where the stem can be cut to reduce its length.

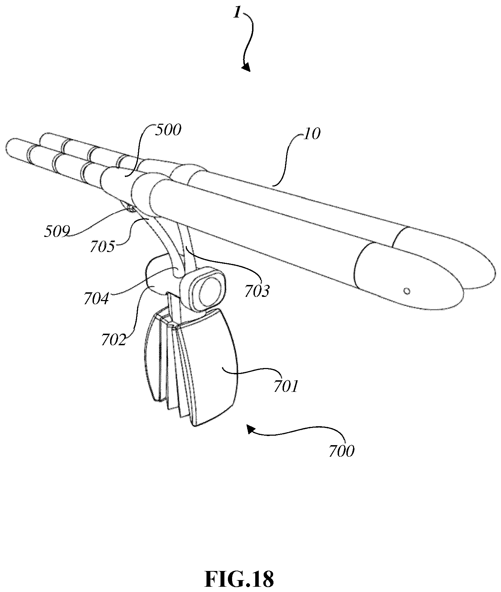

As for the integrated fluid storage and pressurization unit (700), which forms part of the device (1) and is best shown in FIG. 18, it comprises a single reservoir (701) which has a flexible structure in the form of bellows and lateral compression, it allows to contain and pump enough fluid to pressurize each one of the chambers (100) (not illustrated) of each one of the erectable portions (10) of the device (1). The integrated storage and pressurization unit (700) also includes a pressurization control means which comprises a pressure relief valve (702) that establishes controlled fluid communication between the erectable portions (10) of the device (1) and said reservoir (701) in the form of a bellows; where said integrated unit (700) has a size that allows it to be placed in the scrotal sac.

The valve (702) is connected on one side to the reservoir (701) in the form of a bellows and on the other it is connected to a pair of flexible conduits (703) that transfer the fluid between the reservoir (701) and each of the erectable portions (10) of the device (1). Where each of the flexible conduits (703) comprises a lower end (704) for connection to the valve (702) and an opposite upper end (705) for connection to the lower opening (509) of each of the intermediate connectors (500).

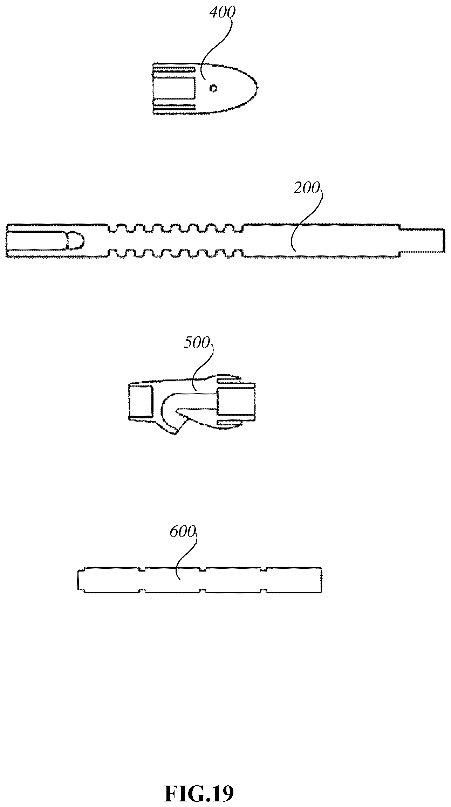

Taking as reference the set of components that appear in FIG. 19, the nerve (200), the point element (400), the intermediate connector (500) and the anchoring stem (600) are made of biocompatible elastomeric material, such as medical grade silicone applied in different thicknesses and hardness for each element.

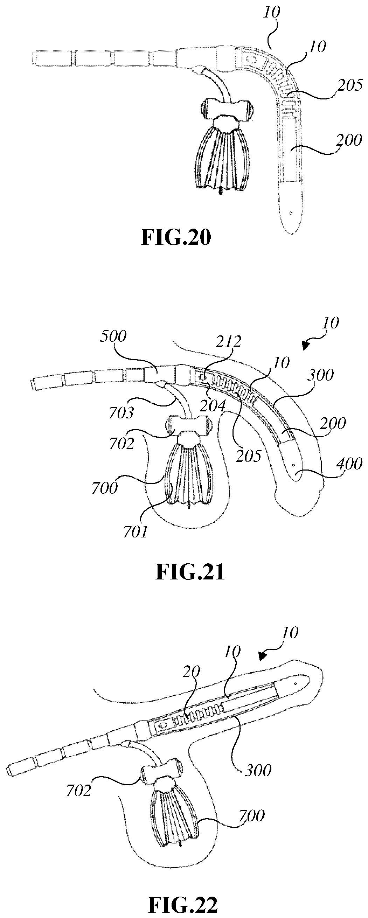

In operation, when the device (1) is in a state of total flaccidity, as exemplified in FIG. 20, the erectable portion (10) of the device remains folded downwards due to the effect of the foldable intermediate section (205) of the nerve (200) and due to the lack of pressurization inside the chamber (100), however, the penis shows a natural flaccid appearance where a certain length and thickness are preserved.

In the erection process, as shown in FIG.21, the user activates the integrated unit (700) performing lateral compressive actions of the reservoir (701) so that its bellows-shaped structure collapses and pushes the fluid out of itself, this allows the fluid to be transferred to the chamber (100) with a single pump and is pressurized by the action of the valve (702) that prevents the return of the fluid to the reservoir (701). The fluid leaves the integrated unit (700) through the flexible conduits (703), enters the intermediate connector (500) where it is transferred to the proximal portion (204) of the nerve (200) where it passes through the inner axial channel (not illustrated) to then exit in an equal and constant manner through each of the lateral openings (212) arranged in the nerve (200); the chamber (100) begins to fill and as its pressurization increases, it stiffens the entire erectable portion (10), the penis begins to rise from the point element (400) changing the axial axis of the nerve (200) from a folded downwards curved state towards a slightly curved inclined arrangement, so that later at higher pressurization, it ends at an upward angle, as it can be seen in FIG. 22; further pressurization of the chamber (100) also causes the axial extension of the collapsible portion (205) of the nerve (200), in conjunction with the axial and radial expansion of the membrane (300); thus, the erectable portion (10) of the device (1) increases its rigidity, increases its length, thickness and changes its position to one of erection, replicating the same changes in the body of the penis which gives a desired effect of obtaining a functional erection in a short time with little manipulation of the integrated unit (700) and with an increase in size and turgidity of the organ.

The deactivation of the erection process is carried out with the opening of the pressure relief valve (702), compressing it laterally in the area of the scrotum; this opening of the valve causes the pressurized fluid in the chamber (100) to begin to return to the reservoir (701) following the same route that it made on its way out, but in the opposite direction.

The invention also includes the production procedure for the prosthetic device (1), which makes it possible to obtain a device of variable length that can be implanted in a wide range of patients depending on the size of their penis, without the production of the device meaning to have special tooling for each measure of each piece;

In a first embodiment, the production procedure of the prosthetic device (1) comprises the steps of:
a) moulding the nerve (200) of a single predefined length with the distal section (206) of exceeded length;
b) moulding the point element (400) with a single axial opening (404) at its proximal end (402);
c) moulding the intermediate connector (500);
d) manufacturing the expandable membrane (300) with a constant diameter and thickness and an indeterminate length as a tubular sleeve;
e) moulding the anchoring stem (600) of a determined diameter and indeterminate length;
f) defining the final length of the nerve (200) cutting its distal end (201);
g) cutting a length of membrane (300) in accordance with the final length of the cut nerve (200), defining a distal end (301) and a proximal end (302) of said cut membrane piece (300);
h) mounting, joining and sealing the joints of all the pieces together;
i) arranging the integrated storage and pressurization unit (700) with a predefined amount of internal fluid according to the final size of the prosthesis and joining its flexible conduits (703) with each of the intermediate connectors (500).

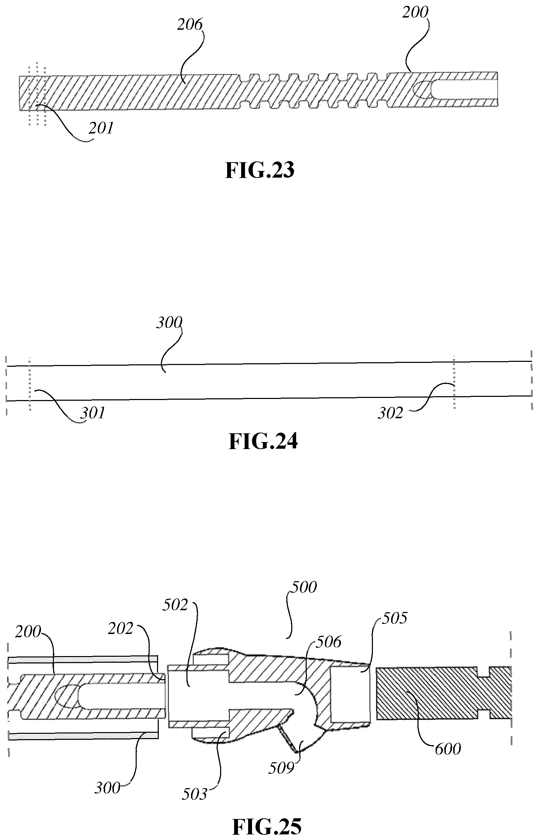

In this embodiment, as illustrated in FIG. 23 and mentioned in step a) of the procedure, the nerve (200) is moulded with a single predefined length with the distal section (206) having an exceeded length, so that subsequently, as mentioned in step f), the final length of the nerve (200) is defined by freely cutting its distal end (201).

Similarly, as indicated in step d), the expandable membrane (300) is manufactured with a constant diameter and thickness and an indeterminate length as a tubular sleeve where this thickness is preferably 1mm. Then, as indicated in step g), a piece of membrane (300) is cut in length according to the final length of the cut nerve (200) defining a distal end (301) and a proximal end (302) in said piece of membrane (300), as illustrated in FIG. 24.

As seen in FIG. 25, when moulding the intermediate connector (500), its central cavity (502) has a diameter that coincides with the outer diameter of the proximal end (202) of the nerve (200); its coaxial slot (503) has a size matching the cross section of the membrane (300); its central recess (505) has a diameter that coincides with the diameter of the anchoring stem (600); and the lower opening (509) of its inner channel (506) has a diameter matching the upper end (705) of the flexible conduit (703) of the integrated storage and pressurization unit (700) (not shown).

The nerve (200), the intermediate connector (500), the point element (400), the anchoring stem (600) and the flexible reservoir (701) of the integrated storage and pressurization unit (700) are moulded in biocompatible elastomeric material preferably a medical grade silicone polymer, in suitable matrices for each part.

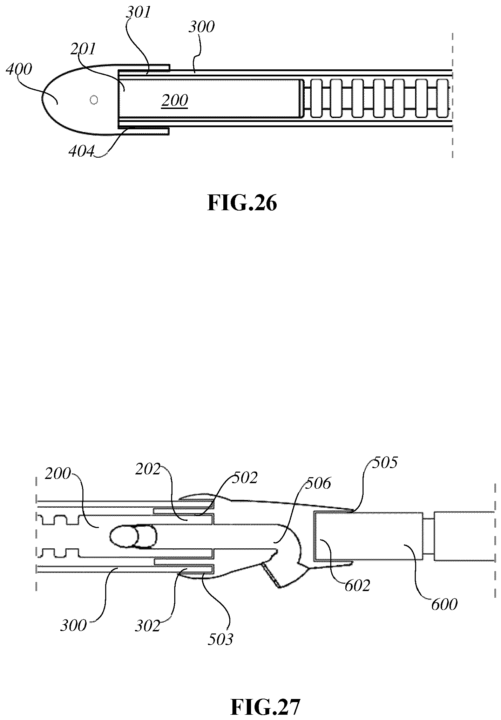

By mounting, joining and sealing all the pieces together, as seen in FIG. 26, the distal end (201) of the nerve (200) and the distal end (301) of the membrane (300) are coupled together and sealed within the central axial opening (404) of the point element (400); while, as seen in FIG. 27, the proximal end (202) of the nerve (200) is coupled and sealed in the central cavity (502) of the connector (500) while the proximal end (302) of the membrane (300) is coupled and sealed to its coaxial annular groove (503); the distal end (602) of the anchoring stem (600) is coupled and sealed within the central recess (505) of the connector (500); and the upper end (705) of the flexible conduit (703) of the integrated unit (700) is coupled and sealed to the lower opening (509) of the connector 500 (not shown).

A second embodiment of the production procedure of the prosthetic device (1) comprises the steps of:
a) moulding the nerve (200) according to at least three different predefined lengths, each with the intermediate section (205) of a length proportional to the total length and with its distal section (206) comprising a perimeter recess (207) at its point;
b) moulding the point element (400) having a central axial opening (404) and an outer annular groove (405);
c) moulding the intermediate connector (500);
d) manufacturing the membrane (300) with a constant diameter and thickness and an indeterminate length as a tubular sleeve;
e) moulding the anchoring stem (600) of a determined diameter and indeterminate length;
f) sizing and cutting a piece of membrane (300) in length according to the length of the moulded nerve (200) defining a distal end (301) and a proximal end (302) of said piece of membrane (300);
g) mounting, joining and sealing the joints of all the pieces together.
h) arranging the integrated storage and pressurization unit (700) with a predefined amount of internal fluid according to the final size of each prosthesis and joining its flexible conduits (703) with each of the intermediate connectors (500).

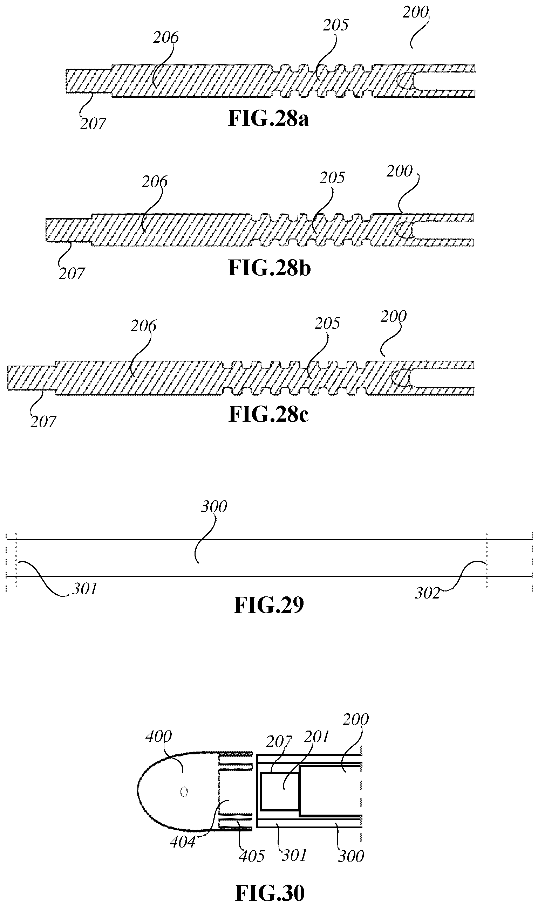

In this second embodiment, as illustrated in FIG. 28a, FIG. 28b and FIG. 28c as well as mentioned in step a) of the procedure, the nerve (200) is moulded according to at least three different predefined lengths, a shorter length, a longer length and an intermediate length; each one with the intermediate section (205) of a length proportional to the total length and with its distal section (206) comprising a perimeter recess (207) at its point. Preferably, the shortest length is 8 centimetres, the intermediate length is 12 centimetres, and the longest length is 16 centimetres.

Similarly, as indicated in step d), the expandable membrane (300) is manufactured with a constant diameter and thickness and an indeterminate length as a tubular sleeve, where this thickness is preferably 1mm. Then, as indicated in step g), a piece of membrane (300) is cut in length according to the length of the moulded nerve (200) defining a distal end (301) and a proximal end (302) in said piece of membrane (300), as illustrated in FIG. 29.

In the same way that it is illustrated in the aforementioned FIG. 25, when moulding the intermediate connector (500), its central cavity (502) has a diameter that coincides with the outer diameter of the proximal end (202) of the nerve (200); its coaxial slot (503) has a size matching the cross section of the membrane (300); its central recess (505) has a diameter that coincides with the diameter of the anchoring stem (600); and the lower opening (509) of its inner channel (506) has a diameter matching the upper end (705) of the flexible conduit (703) of the integrated storage and pressurization unit (700) (not shown).

In this second embodiment of the production procedure, it is also the case that the nerve (200), the intermediate connector (500), the point element (400), the anchoring stem (600) and the flexible reservoir (701) of the integrated (700) storage and pressurization unit are moulded in biocompatible elastomeric material preferably a medical grade silicone polymer, in suitable matrices for each piece.

By mounting, joining and sealing all the pieces together, as seen in FIG. 30, the distal end (201) of the nerve (200) with the perimeter recess (207) is coupled and sealed within the central axial opening (404) of the point element (400); while the distal end (301) of the membrane (300) is coupled and sealed within the outer annular groove (405) of the point element (400). In the same way as illustrated in FIG. 27 above, the proximal end (202) of the nerve (200) is coupled and sealed in the central cavity (502) of the connector (500) while the proximal end (302) of the membrane (300) is coupled and sealed to its coaxial annular groove (503); the distal end (602) of the anchoring stem (600) is coupled and sealed within the central recess (505) of the connector (500); and the upper end 705 of the flexible conduit (703) of the integrated unit (700) is coupled and sealed to the lower opening (509) of the connector (500) (not shown).

## Claims

**1.** Prosthetic device (1) implantable within the cavernous body of a penis with erectile dysfunction and its production procedure, wherein the device is of the type formed by two cylinders that are inflatable with a fluid, it allows to be implanted in a wide range of patients depending on the length of the penis and optimizes the use of a low volume of fluid to achieve a functional erection; **CHARACTERIZED in that** it comprises an erectable portion (10) of variable length composed of a pressurizable chamber (100) with a low volume of fluid formed between an expandable membrane (300) and an axially extensible and foldable longitudinal nerve (200) which extend between a distal tip element (400) and an intermediate connector (500) from which emerges a cuttable anchor rod (600), where said nerve (200) is in one piece that is formed by a distal section (206) of variable length, an intermediate section (205) that is foldable and extensible in the axial direction, and a proximal section (204) that includes optimized means for continuous lateral supply of the fluid towards the pressurizable chamber (100); said device (1) also having an integrated fluid storage and pressurization unit (700) implantable in the scrotal sacs.

**2.** Prosthetic device (1) according to claim 1, **CHARACTERIZED in that** said optimized means of continuous lateral supply of the fluid comprise an internal axial channel (208) in the proximal portion (204) of the nerve (200), which has an open end (209) and a pointed opposite inner end (210) which branches laterally in a horizontal axis (x) of the nerve forming lateral channels (211) that flow into corresponding lateral openings (212), having said lateral channels (211) an inclined path with respect to an axial axis (z) of the nerve (200).

**3.** Prosthetic device (1) according to claim 1, **CHARACTERIZED in that** the nerve (200) is formed by a longitudinal robust body with a continuous outer surface (203), a distal end (201) and a proximal end (202) between which the proximal (204), intermediate (205) and distal (206) sections extend, where the proximal section (204) is partially hollow, the intermediate section (205) has a reduction (213) in its mass and the distal section (206) has a mantle (216) of variable length.

**4.** Prosthetic device (1) according to claim 3, **CHARACTERIZED in that** said distal section (206) has a cuttable distal end (201), to adjust the length of the nerve (200).

**5.** Prosthetic device (1) according to claim 3, **CHARACTERIZED in that** said distal section (206) has different preset lengths and includes a distal perimeter recess (217).

**6.** Prosthetic device (1) according to claim 3, **CHARACTERIZED in that** said intermediate section (205) has an axial core (214) of smaller diameter than the rest of the nerve (200), which in turn, comprises a plurality spaced annular nerves (215) forming a corrugation that folds and extends axially.

**7.** Prosthetic device (1) according to claim 3, **CHARACTERIZED in that** said intermediate section (205) has a microperforated structure that weakens the portion and allows its folding and axial extension.

**8.** Prosthetic device (1) according to claim 3, **CHARACTERIZED in that** said intermediate section (205) has a surface with successive radial depressions forming an accordion shaped portion that allows its folding and axial extension.

**9.** Prosthetic device (1) according to any of the preceding claims, **CHARACTERIZED in that** the intermediate section (205) has a single length.

**10.** Prosthetic device (1) according to any of the preceding claims, **CHARACTERIZED in that** the intermediate section (205) has different preset lengths.

**11.** Prosthetic device (1) according to claim 1, **CHARACTERIZED in that** the expandable membrane (300) is a textile membrane formed by a continuous tubular fabric without longitudinal seam, embedded in medical grade silicone that allows high pressure and rigidity ensuring its expandable characteristic under sealing and impermeability conditions, which comprises an open distal end (301), an open proximal end (302), an inner surface (303) and an outer surface (304).

**12.** Prosthetic device (1) according to claim 11, **CHARACTERIZED in that** the tubular tissue of said membrane (300) is formed by an orthogonal mesh where both the longitudinal and the concentric fibres present a normally corrugated state that allows the expansion of the membrane in both directions.

**12.** Prosthetic device (1) according to claim 11, **CHARACTERIZED in that** the tubular fabric of said membrane (300) is formed by a diagonal mesh that allows the expansion of the membrane in both directions.

**13.** Prosthetic device (1) according to any of the preceding claims, **CHARACTERIZED in that** the membrane (300) is attached to the intermediate connector (500) through its proximal end (302) and is attached to the tip element (400) through its distal end (301).

**14.** Prosthetic device (1) according to claim 1, **CHARACTERIZED in that** the pressurizable chamber (100) is formed by a cylindrical space radially comprised between the outer surface (203) of the nerve (200) and the inner surface (303) of the membrane (300), and axially it has a distal zone (101) that limits with the tip element (400) and a proximal zone (102) that limits with the intermediate connector (500).

**15.** Prosthetic device (1) according to any of the preceding claims, **CHARACTERIZED in that** the pressurizable chamber (100) begins to fill or empty from its proximal area (102) with the fluid that emerges or enters through the lateral openings (212) of the nerve (200).

**16.** Prosthetic device (1) according to claim 1, **CHARACTERIZED in that** the tip element (400) is solid of elastomeric material, it has a rounded distal end (401) and a proximal portion (402) with a straight edge (403) comprising a central axial opening (404).

**17.** Prosthetic device (1) according to claim 1, **CHARACTERIZED in that** the tip element (400) is solid of elastomeric material, it has a rounded distal end (401) and a proximal portion (402) with a straight edge (403) comprising a central axial opening (404) and an outer annular groove (405) coaxial to the central opening (404).

**18.** Prosthetic device (1) according to claim 1, **CHARACTERIZED in that** the intermediate connector (500) is a solid piece with a distal end (501) that has a central cavity (502), an annular groove (503) coaxial to the central cavity (502), a proximal end (504) with a central recess (505) and an inner channel (506).

**19.** Prosthetic device (1) according to claim 19, **CHARACTERIZED in that** said inner channel (506) is curved, it has an open front end (507) that connects with the central cavity (502) and a lower curved end (508) that flows out in a lower opening (509).

**20.** Prosthetic device (1) according to claim 1, **CHARACTERIZED in that** the anchor rod (600) is a preferably cylindrical body, with a cuttable rear end (601) and a front end (602) through which it joins to the intermediate connector (500); it comprises a series of annular grooves (603) regularly spaced from each other that determine cutting points to cut its length.

**21.** Prosthetic device (1) according to claim 1, **CHARACTERIZED in that** the integrated fluid storage and pressurization unit (700) comprises a single reservoir (701) which has a flexible structure in the form of a bellows and lateral compression which allows to contain and pump sufficient fluid to pressurize each one of the pressurizable chambers (100) of each one of the erectable portions (10) of the device (1).

**22.** Prosthetic device (1) according to claim 22, **CHARACTERIZED in that** the integrated storage and pressurization unit (700) also includes a pressurization control means which comprises a pressure relief valve (702) that establishes a controlled fluid communication between the erectable portions (10) of the device (1) and said reservoir (701) in the form of a bellows; where said integrated unit (700) has a size that allows it to be placed in the scrotal sac.

**23.** Prosthetic device (1) according to claim 23, **CHARACTERIZED in that** the valve (702) is connected on one side to the reservoir (701) in the form of a bellows and on the other it is connected to a pair of flexible conduits (703) that transfer the fluid between the reservoir (701) and each of the erectable portions (10) of the device (1).

**24.** Prosthetic device (1) according to any of the preceding claims, **CHARACTERIZED in that** each of the flexible conduits (703) comprises a lower end (704) for connection to the valve (702) and an upper end (705) connection opposite with the lower opening (509) of each of the intermediate connectors (500).

**25.** Prosthetic device (1) according to claim 1, **CHARACTERIZED in that** the nerve (200), the tip element (400), the intermediate connector (500) and the anchor rod (600) are made of biocompatible elastomeric material, such as a medical grade silicone polymer, applied in different thicknesses and hardness for each element.

**26.** Production procedure of the prosthetic device (1) described between claims 1 to 25, which allows to obtain a device of variable length that can be implanted in a wide range of patients according to the size of their penis, **CHARACTERIZED in that** it comprises the steps of:
a) moulding the nerve (200) of a single predefined length with the distal section (206) of excess length;
b) moulding the tip element (400) with a single axial opening (404) at its proximal end (402);
c) moulding the intermediate connector (500);
d) manufacturing the membrane (300) with a constant diameter and thickness, and an undetermined length as a tubular sleeve;
e) moulding the anchor rod (600) of a determined diameter and undetermined length;
f) defining the final length of the nerve (200) cutting its distal end (201);
g) sizing and cutting a piece of membrane (300) in a length according to the final length of the cut nerve (200), defining a distal end (301) and a proximal end (302) of said piece of membrane (300) already cut;
h) mounting, joining and sealing the joints of all the pieces together;
i) provide the integrated storage and pressurization unit (700) with a predefined amount of internal fluid according to the final size of the prosthesis and join its flexible ducts (703) with each of the intermediate connectors (500).

**27.** Production procedure of the prosthetic device (1) according to claim 26, **CHARACTERIZED in that** when moulding the intermediate connector (500), its central cavity (502) of the distal end (501) has a diameter that coincides with the outer diameter of the proximal end (202) of the nerve (200); its coaxial slot (503) has a size matching the cross section of the tubular membrane (300); its central recess (505) on the proximal face (504) has a diameter that coincides with the diameter of the anchor rod (600); and the lower opening (509) of its inner channel (506) has a diameter that coincides with the upper end (705) of the flexible conduit (703) of the integrated storage and pressurization unit (700).

**28.** Production procedure of the prosthetic device (1) according to claim 26, **CHARACTERIZED in that** the membrane (300) preferably has a thickness of approximately 1mm.

**29.** Production procedure of the prosthetic device (1) according to claim 26, **CHARACTERIZED in that** the nerve (200), the intermediate connector (500), the tip element (400), the anchor rod (600) and the flexible reservoir (701) of the integrated storage and pressurization unit (700) are molded in biocompatible elastomeric material preferably a medical grade silicone polymer, in suitable matrices for each piece.

**30.** Production procedure of the prosthetic device (1) according to claim 26, CHARACTERIZED because when mounting, joining and sealing all the pieces together the distal end (201) of the nerve (200) and the distal end (301) of the tubular membrane (300) are coupled together and sealed within the central axial opening (404) of the tip element (400); the proximal end (202) of the nerve (200) is coupled and sealed in the central cavity (502) of the connector (500) while the proximal end (302) of the membrane (300) is coupled and sealed to the coaxial annular groove (503) of the connector (500); the distal end (602) of the anchor rod (600) is coupled and sealed within the central recess (505) of the connector (500); and the upper end (705) of the flexible conduit (703) of the integrated unit (700) is coupled and sealed to the lower opening (509) of the connector (500).

**31.** Production procedure of the prosthetic device (1) described between claims 1 to 25 which allows to obtain a device of variable length that can be implanted in a wide range of patients according to the size of their penis, **CHARACTERIZED in that** it comprises the steps of:
a) moulding the nerve (200) according to at least three different predefined lengths, each with the intermediate section (205) of a length proportional to the total length and with its distal section (206) comprising a perimeter recess (207) at its tip;
b) moulding the tip element (400) having a central axial opening (404) and an outer annular groove (405);
c) moulding the intermediate connector (500);
d) manufacturing the membrane (300) with a constant diameter and thickness, and an undetermined length as a tubular sleeve;
e) moulding the anchor rod (600) of a determined diameter and undetermined length;
f) sizing and cutting a piece of membrane (300) in length according to the length of the moulded nerve (200) defining a distal end (301) and a proximal end (302) of said piece of membrane (300);
g) mounting, joining and sealing the joints of all the pieces together.
h) providing the integrated storage and pressurization unit (700) with a predefined amount of internal fluid according to the final size of each prosthesis and joining its flexible ducts (703) with each of the intermediate connectors (500).

**32.** Production procedure of the prosthetic device (1) according to claim 31, **CHARACTERIZED in that** the at least three different predefined lengths of the nerve (200) correspond to a shorter length, a longer length and an intermediate length.

**33.** Production procedure of the prosthetic device (1) according to claim 32, **CHARACTERIZED in that** preferably the shortest length is 8 centimetres, the intermediate length is 12 centimetres and the longest length is 16 centimetres.

**34.** Production procedure of the prosthetic device (1) according to claim 31, **CHARACTERIZED in that** when moulding the tip element (400) its central axial opening (404) has a diameter equivalent to the diameter of the lowered end (207) of the nerve (200) and its outer annular groove (405) has a cross section matching the cross section of the membrane (300).

**35.** Production procedure of the prosthetic device (1) according to claim 31, **CHARACTERIZED in that** when moulding the intermediate connector (500) its central cavity (502) of the distal end (501) has a diameter that coincides with the outer diameter of the proximal end (202) of the nerve (200); its coaxial slot (503) has a size matching the cross section of the membrane (300); its central recess (505) on the proximal face (504) has a diameter that coincides with the diameter of the anchor rod (600); and the lower opening (509) of its inner channel (506) has a diameter that coincides with the upper end (705) of the flexible conduit (703) of the integrated storage and pressurization unit (700).

**36.** Production procedure of the prosthetic device (1) according to claim 31, **CHARACTERIZED in that** the membrane (300) is preferably manufactured with a thickness of approximately 1mm.

**37.** Production procedure of the prosthetic device (1) according to claim 31, **CHARACTERIZED in that** the nerve (200), the intermediate connector (500), the tip element (400), the anchor rod (600) and the flexible reservoir (701) of the integrated storage and pressurization unit (700) are molded in biocompatible elastomeric material preferably a medical grade silicone polymer in suitable matrices for each piece.

**38.** Production procedure of the prosthetic device (1) according to claim 31, **CHARACTERIZED in that** when mounting, joining and sealing all the pieces together, the distal end (201) with the perimeter recess (207) of the nerve (200) is coupled and sealed within the central axial opening (404) of the tip element (400); the distal end (301) of the membrane (300) is couped and sealed within the outer annular groove (405) of the tip element (400); the proximal end (202) of the nerve (200) is coupled and sealed in the central cavity (502) of the intermediate connector (500) while the proximal end (302) of the membrane (300) is coupled and sealed in the coaxial annular groove (503) of the connector (500); the distal end (602) of the anchor rod (600) is coupled and sealed within the central recess (505) of the connector (500); and the upper end (705) of the flexible conduit (703) of the integrated unit (700) is coupled and sealed to the lower opening (509) of the connector (500).

## Amended claims

### Amended claims under Art. 19.1 PCT

1. Prosthetic device (1) implantable within the cavernous body of a penis with erectile dysfunction and its production procedure where the device is of the type formed by two cylinders that are inflatable with a fluid allowing it to be implanted in a wide range of patients depending on the length of the penis and optimizes the use of a low volume of fluid to achieve a functional erection; **CHARACTERIZED in that** it comprises an erectable portion (10) of variable length composed of a pressurizable chamber (100) with a low volume of fluid formed between an expandable membrane (300) and an axially extensible and foldable longitudinal nerve (200) which extend between a distal tip element (400) and an intermediate connector (500) from which emerges a cuttable anchor rod (600) , where said nerve (200) is in one piece that is formed by a distal section (206) of variable length, an intermediate section (205) that is foldable and extensible in the axial direction, and a proximal section (204) that includes optimized means for continuous lateral supply of the fluid towards the pressurizable chamber (100); said device (1) also having an integrated fluid storage and pressurization unit (700) implantable in the scrotal sacs.

2. Prosthetic device (1) according to claim 1, **CHARACTERIZED in that** said optimized means of continuous lateral supply of the fluid comprise an internal axial channel (208) in the proximal portion (204) of the nerve (200), which has an open end (209) and a pointed opposite inner end (210) which branches laterally in a horizontal axis (x) of the nerve forming lateral channels (211) that flow into corresponding lateral openings (212), having said lateral channels (211) an inclined path with respect to an axial axis (z) of the nerve (200).

3. Prosthetic device (1) according to claim 1, **CHARACTERIZED in that** the nerve (200) is formed by a longitudinal robust body with a continuous outer surface (203), a distal end (201) and a proximal end (202) between which the proximal (204), intermediate (205) and distal (206) sections extend, where the proximal section (204) is partially hollow, the intermediate section (205) has a reduction (213) in its mass and the distal section (206) has a mantle (216) of variable length.

4. Prosthetic device (1) according to claim 3, **CHARACTERIZED in that** said distal section (206) has a cuttable distal end (201), to adjust the length of the nerve (200).

5. Prosthetic device (1) according to claim 3, **CHARACTERIZED in that** said distal section (206) has different preset lengths and includes a distal perimeter recess (217).

6. Prosthetic device (1) according to claim 3, **CHARACTERIZED in that** said intermediate section (205) has an axial core (214) of smaller diameter than the rest of the nerve (200), which in turn, comprises a plurality spaced annular nerves (215) forming a corrugation that folds and extends axially.

7. Prosthetic device (1) according to claim 3, **CHARACTERIZED in that** said intermediate section (205) has a microperforated structure that weakens the portion and allows its folding and axial extension.

8. Prosthetic device (1) according to claim 3, **CHARACTERIZED in that** said intermediate section (205) has a surface with successive radial depressions forming an accordion shaped portion that allows its folding and axial extension.

9. Prosthetic device (1) according to any of the preceding claims, **CHARACTERIZED in that** the intermediate section (205) has a single length.

10. Prosthetic device (1) according to any of the preceding claims, **CHARACTERIZED in that** the intermediate section (205) has different preset lengths.

11. Prosthetic device (1) according to claim 1, **CHARACTERIZED in that** the expandable membrane (300) is a textile membrane formed by a continuous tubular fabric without longitudinal seam, embedded in medical grade silicone that allows high pressure and rigidity ensuring its expandable characteristic under sealing and impermeability conditions, which comprises an open distal end (301), an open proximal end (302), an inner surface (303) and an outer surface (304).

12. Prosthetic device (1) according to claim 11, **CHARACTERIZED in that** the tubular tissue of said membrane (300) is formed by an orthogonal mesh where both the longitudinal and the concentric fibres present a normally corrugated state that allows the expansion of the membrane in both directions.

13. Prosthetic device (1) according to claim 11, **CHARACTERIZED in that** the tubular fabric of said membrane (300) is formed by a diagonal mesh that allows the expansion of the membrane in both directions.

14. Prosthetic device (1) according to any of the preceding claims, **CHARACTERIZED in that** the membrane (300) is attached to the intermediate connector (500) through its proximal end (302) and is attached to the tip element (400) through its distal end (301).

15. Prosthetic device (1) according to claim 1, **CHARACTERIZED in that** the pressurizable chamber (100) is formed by a cylindrical space radially comprised between the outer surface (203) of the nerve (200) and the inner surface (303) of the membrane (300), and axially it has a distal zone (101) that limits with the tip element (400) and a proximal zone (102) that limits with the intermediate connector (500).

16. Prosthetic device (1) according to any of the preceding claims, **CHARACTERIZED in that** the pressurizable chamber (100) begins to fill or empty from its proximal area (102) with the fluid that emerges or enters through the lateral openings (212) of the nerve (200).

17. Prosthetic device (1) according to claim 1, **CHARACTERIZED in that** the tip element (400) is solid of elastomeric material, it has a rounded distal end (401) and a proximal portion (402) with a straight edge (403) comprising a central axial opening (404).

18. Prosthetic device (1) according to claim 1, **CHARACTERIZED in that** the tip element (400) is solid of elastomeric material, it has a rounded distal end (401) and a proximal portion (402) with a straight edge (403) comprising a central axial opening (404) and an outer annular groove (405) coaxial to the central opening (404).

19. Prosthetic device (1) according to claim 1, **CHARACTERIZED in that** the intermediate connector (500) is a solid piece with a distal end (501) that has a central cavity (502), an annular groove (503) coaxial to the central cavity (502), a proximal end (504) with a central recess (505) and an inner channel (506).

20. Prosthetic device (1) according to claim 19, **CHARACTERIZED in that** said inner channel (506) is curved, it has an open front end (507) that connects with the central cavity (502) and a lower curved end (508) that flows out in a lower opening (509).

21. Prosthetic device (1) according to claim 1, **CHARACTERIZED in that** the anchor rod (600) is a preferably cylindrical body, with a cuttable rear end (601) and a front end (602) through which it joins to the intermediate connector (500); it comprises a series of annular grooves (603) regularly spaced from each other that determine cutting points to cut its length.

22. Prosthetic device (1) according to claim 1, **CHARACTERIZED in that** the integrated fluid storage and pressurization unit (700) comprises a single reservoir (701) which has a flexible structure in the form of a bellows and lateral compression which allows to contain and pump sufficient fluid to pressurize each one of the pressurizable chambers (100) of each one of the erectable portions (10) of the device (1).

23. Prosthetic device (1) according to claim 22, **CHARACTERIZED in that** the integrated storage and pressurization unit (700) also includes a pressurization control means which comprises a pressure relief valve (702) that establishes a controlled fluid communication between the erectable portions (10) of the device (1) and said reservoir (701) in the form of a bellows; where said integrated unit (700) has a size that allows it to be placed in the scrotal sac.

24. Prosthetic device (1) according to claim 23, **CHARACTERIZED in that** the valve (702) is connected on one side to the reservoir (701) in the form of a bellows and on the other it is connected to a pair of flexible conduits (703) that transfer the fluid between the reservoir (701) and each of the erectable portions (10) of the device (1).

25. Prosthetic device (1) according to any of the preceding claims, **CHARACTERIZED in that** each of the flexible conduits (703) comprises a lower end (704) for connection to the valve (702) and an upper end (705) connection opposite with the lower opening (509) of each of the intermediate connectors (500).

26. Prosthetic device (1) according to claim 1, **CHARACTERIZED in that** the nerve (200), the tip element (400), the intermediate connector (500) and the anchor rod (600) are made of biocompatible elastomeric material, such as a medical grade silicone polymer, applied in different thicknesses and hardness for each element.

27. Production procedure of the prosthetic device (1) described between claims 1 to 26, which allows to obtain a device of variable length that can be implanted in a wide range of patients according to the size of their penis, **CHARACTERIZED in that** it comprises the steps of:
a) moulding the nerve (200) of a single predefined length with the distal section (206) of excess length;
b) moulding the tip element (400) with a single axial opening (404) at its proximal end (402);
c) moulding the intermediate connector (500);
d) manufacturing the membrane (300) with a constant diameter and thickness, and an undetermined length as a tubular sleeve;
e) moulding the anchor rod (600) of a determined diameter and undetermined length;
f) defining the final length of the nerve (200) cutting its distal end (201);
g) sizing and cutting a piece of membrane (300) in a length according to the final length of the cut nerve (200), defining a distal end (301) and a proximal end (302) of said piece of membrane (300) already cut;
h) mounting, joining and sealing the joints of all the pieces together;
i) provide the integrated storage and pressurization unit (700) with a predefined amount of internal fluid according to the final size of the prosthesis and join its flexible ducts (703) with each of the intermediate connectors (500).

28. Production procedure of the prosthetic device (1) according to claim 27, **CHARACTERIZED in that** when moulding the intermediate connector (500), its central cavity (502) of the distal end (501) has a diameter that coincides with the outer diameter of the proximal end (202) of the nerve (200); its coaxial slot (503) has a size matching the cross section of the tubular membrane (300); its central recess (505) on the proximal face (504) has a diameter that coincides with the diameter of the anchor rod (600); and the lower opening (509) of its inner channel (506) has a diameter that coincides with the upper end (705) of the flexible conduit (703) of the integrated storage and pressurization unit (700).

29. Production procedure of the prosthetic device (1) according to claim 27, **CHARACTERIZED in that** the membrane (300) preferably has a thickness of approximately 1mm.

30. Production procedure of the prosthetic device (1) according to claim 27, **CHARACTERIZED in that** the nerve (200), the intermediate connector (500), the tip element (400), the anchor rod (600) and the flexible reservoir (701) of the integrated storage and pressurization unit (700) are moulded in biocompatible elastomeric material preferably a medical grade silicone polymer, in suitable matrices for each piece.

31. Production procedure of the prosthetic device (1) according to claim 27, CHARACTERIZED because when mounting, joining and sealing all the pieces together the distal end (201) of the nerve (200) and the distal end (301) of the tubular membrane (300) are coupled together and sealed within the central axial opening (404) of the tip element (400); the proximal end (202) of the nerve (200) is coupled and sealed in the central cavity (502) of the connector (500) while the proximal end (302) of the membrane (300) is coupled and sealed to the coaxial annular groove (503) of the connector (500); the distal end (602) of the anchor rod (600) is coupled and sealed within the central recess (505) of the connector (500); and the upper end (705) of the flexible conduit (703) of the integrated unit (700) is coupled and sealed to the lower opening (509) of the connector (500).

32. Production procedure of the prosthetic device (1) described between claims 1 to 26 which allows to obtain a device of variable length that can be implanted in a wide range of patients according to the size of their penis, **CHARACTERIZED in that** it comprises the steps of:
a) moulding the nerve (200) according to at least three different predefined lengths, each with the intermediate section (205) of a length proportional to the total length and with its distal section (206) comprising a perimeter recess (207) at its tip;
b) moulding the tip element (400) having a central axial opening (404) and an outer annular groove (405);
c) moulding the intermediate connector (500);
d) manufacturing the membrane (300) with a constant diameter and thickness, and an undetermined length as a tubular sleeve;
e) moulding the anchor rod (600) of a determined diameter and undetermined length;
f) sizing and cutting a piece of membrane (300) in length according to the length of the moulded nerve (200) defining a distal end (301) and a proximal end (302) of said piece of membrane (300);
g) mounting, joining and sealing the joints of all the pieces together.
h) providing the integrated storage and pressurization unit (700) with a predefined amount of internal fluid according to the final size of each prosthesis and joining its flexible ducts (703) with each of the intermediate connectors (500).

33. Production procedure of the prosthetic device (1) according to claim 32, **CHARACTERIZED in that** the at least three different predefined lengths of the nerve (200) correspond to a shorter length, a longer length and an intermediate length.

34. Production procedure of the prosthetic device (1) according to claim 33, **CHARACTERIZED in that** preferably the shortest length is 8 centimetres, the intermediate length is 12 centimetres and the longest length is 16 centimetres.

35. Production procedure of the prosthetic device (1) according to claim 32, **CHARACTERIZED in that** when moulding the tip element (400) its central axial opening (404) has a diameter equivalent to the diameter of the lowered end (207) of the nerve (200) and its outer annular groove (405) has a cross section matching the cross section of the membrane (300).

36. Production procedure of the prosthetic device (1) according to claim 32, **CHARACTERIZED in that** when moulding the intermediate connector (500) its central cavity (502) of the distal end (501) has a diameter that coincides with the outer diameter of the proximal end (202) of the nerve (200); its coaxial slot (503) has a size matching the cross section of the membrane (300); its central recess (505) on the proximal face (504) has a diameter that coincides with the diameter of the anchor rod (600); and the lower opening (509) of its inner channel (506) has a diameter that coincides with the upper end (705) of the flexible conduit (703) of the integrated storage and pressurization unit (700).

37. Production procedure of the prosthetic device (1) according to claim 32, **CHARACTERIZED in that** the membrane (300) is preferably manufactured with a thickness of approximately 1mm.

38. Production procedure of the prosthetic device (1) according to claim 32, **CHARACTERIZED in that** the nerve (200), the intermediate connector (500), the tip element (400), the anchor rod (600) and the flexible reservoir (701) of the integrated storage and pressurization unit (700) are moulded in biocompatible elastomeric material preferably a medical grade silicone polymer in suitable matrices for each piece.

39. Production procedure of the prosthetic device (1) according to claim 32, **CHARACTERIZED in that** when mounting, joining and sealing all the pieces together, the distal end (201) with the perimeter recess (207) of the nerve (200) is coupled and sealed within the central axial opening (404) of the tip element (400); the distal end (301) of the membrane (300) is couped and sealed within the outer annular groove (405) of the tip element (400); the proximal end (202) of the nerve (200) is coupled and sealed in the central cavity (502) of the intermediate connector (500) while the proximal end (302) of the membrane (300) is coupled and sealed in the coaxial annular groove (503) of the connector (500); the distal end (602) of the anchor rod (600) is coupled and sealed within the central recess (505) of the connector (500); and the upper end (705) of the flexible conduit (703) of the integrated unit (700) is coupled and sealed to the lower opening (509) of the connector (500).
